(19) 

**Europäisches Patentamt**
**European Patent Office**
**Office européen des brevets**

(11) **EP 4 243 890 B1**

(12) **EUROPEAN PATENT SPECIFICATION**

(45) Date of publication and mention
of the grant of the patent:
**29.04.2026 Bulletin 2026/18**

(21) Application number: **21820401.4**

(22) Date of filing: **11.11.2021**

(51) International Patent Classification (IPC):
**A61M 1/00** (2006.01)

(52) Cooperative Patent Classification (CPC):
**A61M 1/73; A61M 1/60; A61M 1/65; A61M 1/79;**
A61M 2205/3313; A61M 2205/3389;
A61M 2205/7545; A61M 2209/084

(86) International application number:
**PCT/US2021/058891**

(87) International publication number:
**WO 2022/103912 (19.05.2022 Gazette 2022/20)**

(54) **QUANTIFYING BLOOD LOSS WITH A MEDICAL WASTE COLLECTION SYSTEM**

QUANTIFIZIERUNG VON BLUTVERLUST MIT EINEM SYSTEM ZUR SAMMLUNG
MEDIZINISCHER ABFÄLLE

QUANTIFICATION DE LA PERTE DE SANG AVEC UN SYSTÈME DE COLLECTE DE DÉCHETS
MÉDICAUX

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.11.2020 US 202063112382 P**

(43) Date of publication of application:
**20.09.2023 Bulletin 2023/38**

(73) Proprietor: **Stryker Corporation
Portage, MI 49002-9711 (US)**

(72) Inventors:
• **FAUL, Stephen
Cork T12 H5CC (IE)**
• **VANDERWOUDE, Brian James
Portage, MI 49002 (US)**
• **MACLACHLAN, Brian
Norton Shores, MI 49444 (US)**

• **BUCKLEY, Kevin
Cobh, Co. Cork (IE)**
• **LADUKE, Peter
Holland, MI 49424 (US)**
• **NUNAN, Gerard
Cork Co. Cork, P31 F761 (IE)**
• **ZOLLINGER, Michael
Chelsea, MI 48118 (US)**

(74) Representative: **Röthinger, Rainer
Wuesthoff & Wuesthoff
Patentanwälte und Rechtsanwalt PartG mbB
Schweigerstrasse 2
81541 München (DE)**

(56) References cited:
**WO-A1-2016/183290      US-A1- 2016 367 734
US-A1- 2020 061 255      US-A1- 2020 324 028**

## Description

PRIORITY CLAIM

This application claims priority to and all the benefits of United States Provisional Patent Application No. 63/112,382, filed November 11, 2020.

BACKGROUND

A byproduct of some surgical procedures is the generation of liquid, semisolid, and/or solid waste material. The liquid waste material may include bodily fluids and irrigating solution(s) at the surgical site, and the solid and semisolid waste material may include bits of tissue and pieces of surgical material(s). The medical waste, regardless of its phase, is preferably collected so it neither fouls the surgical site nor becomes a biohazard in the medical suite in which the procedure is being performed.

The medical waste may be removed from the surgical site through a suction tube under the influence of a vacuum provided by a medical waste collection system. One exemplary medical waste collection system is sold under the tradename Neptune by Stryker Corporation (Kalamazoo, Mich.) with certain versions of the medical waste collection system disclosed in commonly-owned United States Patent Publication No. 2005/0171495, published August 4, 2005, International Publication No. WO 2007/070570, published June 21, 2007, and International Publication No. WO 2014/066337, published May 1, 2014. A manifold may be provided that facilitates interfacing the suction tube with the medical waste collection system. The manifold may be disposable.

The collected liquid waste material may include blood, and the blood may be in a suction path with other bodily fluids such as interstitial fluid, mucus, and the like. Determining blood loss during surgery may be used to monitor patient health. Excessive blood loss may be indicative of surgical complications, and determining blood loss facilitates assessing transfusion requirements. Of particular interest is childbirth, wherein obstetric hemorrhage is a major cause of maternal morbidity - eleven percent of maternal deaths in the United States reportedly being caused by postpartum hemorrhage. Earlier detection of obstetric hemorrhage may significantly reduce the maternal morbidity rate. There has been a growing impetus among clinicians and governing bodies to increase the usage and accuracy of blood loss quantification methods and tools, especially for vaginal and Caesarean deliveries where postpartum hemorrhage is a of vital concern.

It is known to estimate blood loss during surgery by visual evaluation of absorbent articles (e.g., sponges, surgical gowns, bedding, or drapes), measurements of the absorbent articles using a scale, and/or graduated collection vessels underneath the operating table. Additionally or alternatively, an estimation of blood loss may include visually observing the color of the blood and non-blood mixture in a waste container after being suctioned from the surgical site.

The aforementioned methods may provide suboptimal accuracy, and with the aforementioned methods there may be appreciable delay between the determination of the blood loss and the blood loss itself. Therefore, it would be desirable to provide improved system, devices, and methods for quantifying blood loss during the surgical procedure in an accurate and rapid manner.

WO 2016/183290 A1, US 2020/324028 A1, US 2020/061255 A1 and US 2016/367734 A1 disclose further relevant prior art. WO 2016/183290 A1 is the closest prior art.

SUMMARY

With the scope of the invention defined by the claims, the present disclosure is directed to performing quantitative blood loss analysis with a medical waste collection system and/or a manifold. The medical waste collection system includes at least one waste container defining a waste volume for collecting and storing the waste material. A vacuum source is configured to draw suction on the waste container. A control panel is in communication with a controller including a processor. The controller is configured to operate the vacuum regulator to adjust the vacuum level in the waste container. The medical waste collection system includes at least one receiver sized to removably receive at least a portion of a manifold.

A fluid characterization module is configured to facilitate quantifying a concentration of blood within the fluid being draw through the medical waste collection system under the influence of suction. The fluid characterization module includes a sensor assembly, and further may include a module housing. The fluid characterization module may be free-floating and coupled to a dongle, or integrated with the receiver. The sensor assembly includes emitter(s) and sensor(s). The emitters are configured to emit energy, and the sensors are configured to detect the emitted energy. The emitters may be light emitting diodes (LEDs) and the sensors being photodetectors. The emitters and the sensors may be configured to be positioned opposite the detection window. The first emitter may be an infrared LED, and the second emitter may be a visible-light LED. The infrared LED may be configured to emit light having a wavelength approximately in the range of 700 nanometers (nm) to 1000 nm, and more particularly within the range of 750 nm to 850 nm, and even more particularly within the range of 770 nm to 810 nm. The visible-light LED may be configured to emit light having a wavelength approximately in the range of 400 nm to 600 nm, and more particularly within the range of 550 nm to 600 nm, and even more particularly within the range of 570 nm to 580 nm. The visible-light LED may be a green LED. The sensors detect the emitted light, and more particularly the light after being transmitted or scattered through the fluid passing through the detection window. The detected intensity of the transmitted light or the scattered light may be indicative of the transmissivity, opacity, and/or other physical property of the fluid. The four measurements - two of transmitted light and two of scattered light - are values provided to the processor to execute an algorithm to determine the concentration of blood of the fluid passing through the detection window.

The manifold includes a housing that defines a manifold volume. The manifold may include a head coupled to a trunk that cooperate to define the manifold volume. The head may include an inlet fitting configured to removably receive at least one suction tube. The trunk may define an outlet opening in fluid communication with the manifold volume and the inlet fitting. A seal may be coupled to the housing and sized to cover the outlet opening. A filter element may be disposed within the manifold volume. The outlet opening may be offset from a longitudinal axis of the manifold and configured to function as a valve driver of a rotatable valve of the medical waste collection system. The manifold may include a body portion, a first leg, and/or a second leg extend proximally from the body portion. The first and second legs may be spaced apart from one another by a void. The manifold may include an arm, a lock element, a spine, and/or a catch. A rim may be disposed on the first leg and define an outlet opening. The arms each include a proximally-directed surface positioned distal to the rim. A distally-directed surface of the catches may be positioned proximal to the rim, and positioned proximal to the proximally-directed surfaces of the arms. A proximally-directed surface of the spine may be positioned distal to the rim, positioned distal to the distally-directed surfaces of the catches, and positioned distal to the proximally-directed surfaces of the arms. A distally-directed surface of the lock elements may be positioned distal to the rim, positioned distal to the distally-directed surfaces of the catches, positioned distal to the proximally-directed surfaces of the arms, and positioned distal to the proximally-directed surface of the spine. All internal features of the manifold may be included with the trunk with the offset outlet opening or the trunk with the first and second legs.

At least a portion of the housing of the manifold is optically clear to define a detection window. The detection window is configured to be positioned adjacent or between the emitters and the sensors of the sensor assembly. The emitter and the sensor of the sensor assembly are configured to detect an optical characteristic of the fluid passing through the detection window. The portion of the housing defining the detection window may be formed from transparent material. The manifold may include a projection. The projection may be disposed on the and extend longitudinally in the proximal-to-distal direction. The projection may include a coupling feature configured to engage the module housing of the fluid characterization module. The coupling feature may be a rail be sized to be slidably positioned with a slot defined by the module housing. The projection may define a sump configured to facilitate the gas in the fluid separating from the liquid in the fluid prior to the fluid characterization module measuring the transmitted light and the scattered light. The sump may be positioned below the manifold volume. The accumulation of the fluid within the sump on provides a brief period of time during which the gas may separate from the liquid.

The manifold may further a fluid director disposed within the housing. The fluid director includes various geometries configured to provide a torturous path to the fluid within the manifold. The fluid director is disposed within the manifold volume, and may be at least partially disposed within the head. The geometries may cooperate with internal geometries of the housing to define a fluid flow path, a liquid flow path, and a gas flow path. The gas flow path may be positioned near an upper aspect of the manifold, and the liquid flow path may be positioned near a lower aspect of the manifold. The liquid flow path may be at least partially defined by the sump that includes the detection window. The fluid director may include a first barrier and a second barrier, and define a gas inlet, a gas channel, a liquid channel, and a fluid outlet. The second barrier and the housing of the manifold may cooperate to define the liquid inlet between the manifold volume and the sump. The first barrier is configured to impart the tortuous path to the fluid entering the manifold through the inlet fitting. The separated gas is suctioned through the gas channel and through the fluid outlet to pass through the filter element and the outlet opening. The separated liquid may be simultaneously suctioned through the liquid inlet from the vacuum provided by the medical waste collection system. The separated liquid may be further is suctioned through the sump including the detection window, the liquid channel, and the fluid outlet. The liquid flow path and the gas flow path may be joined prior to the fluid outlet.

The fluid director may be positioned proximal to the filter element. The fluid director may define the detection window. The fluid characterization module may be disposed within the manifold. The fluid director may include at least one redirecting aperture in fluid communication with a lateral channel extending longitudinally within the manifold. The fluid director may define a central channel in communication with the sump, and the gas inlet near a proximal end of the manifold. The filter element may be non-cylindrical and positioned in a stacked arrangement with the fluid director. The filter element may be hemicylindrical with a flat surface arranged to be supported atop an upper surface of the fluid director.

The manifold may include a second filter element. The second filter element may be disposed within the sump. A straw may be at least partially disposed within the sump. The vacuum provided by the system is drawn through the straw to draw the liquid from the sump through the first end of the straw against the force of gravity. The liquid is drawn through the straw, and further through the detection window defined by a second projection. The straw may extend through the second filter element.

The head may define an accessory sleeve extending from an accessory opening. A first inlet fitting may extend upwardly from an upper barrier of the accessory sleeve. The accessory sleeve is in fluid communication with the manifold volume. The fluid characterization module is configured to be removably positioned through the accessory opening and supported within the accessory sleeve. A tray may facilitate the removable positioning of the fluid characterization module within the accessory sleeve to be in optical communication with the suction path, and in particular the inflow of the fluid through the first inlet fitting. The fluid characterization module may include a printed circuit board (PCB) assembly sized and shaped to an opening of a cavity of the tray. The fluid characterization module includes the sensor assembly. The fluid characterization module may further include one or more of an LED driver integrated circuit, a photosensor integrated circuit, and a microcontroller in communication with the LED driver integrated circuit and the photosensor integrated circuit, and a communication module, a battery, and a battery management integrated circuit.

A radiofrequency identification (RFID) tag may be coupled to the manifold and positioned to be detected by a data reader of the medical waste collection system. The RFID tag transmits the data from its memory to the data reader, and the controller of the medical waste collection system performs a consequent action. The memory of the RFID tag may store calibration data for the emitters and/or the sensors.

The fluid characterization module may be integrated with the receiver. The fluid characterization module may be disposed on an inlet mechanism of the receiver. The inlet mechanism includes a suction fitting configured to penetrate the seal to be at least partially positioned within the manifold. The emitters and the sensors may be coupled to the inlet mechanism be positioned relative to the detection window on the first leg of the trunk. Implementations of the fluid characterization module may be used alone or in combination. The manifold and the receiver may accommodate a separate fluid characterization module. The output from each of fluid characterization module may be compared and/or combined with one another via the controller or processor to assess or improve accuracy of the determined of the blood concentration within the fluid. The output from one of the fluid characterization modules may be used to facilitate calibration of another one of the fluid characterization modules.

The fluid characterization module may provide for adjustment the gain of one or both of the sensors. As the light detected by the sensors falls below a predetermined transmissivity threshold, the gain of the sensors may be increased. As the light detected by the sensors rises above the predetermined transmissivity threshold, the gain of the sensors may be decreased. The sensor assembly may include additional sensors that are selectively operated based on the detected light transmissivity. The brightness of the emitters may be adjusted based on the detected light transmissivity.

A blood management system may include the medical waste collection system, a sponge system, and a user interface. The data may be forwarded to the electronic medical record (EMR) of the patient. The medical waste collection system may perform the QBL analysis, and transmits the blood volume data wirelessly to the user interface. Additionally or alternatively, another device such as a mobile device, or a remote server or the like, may receive the data described herein and execute the algorithm to perform the QBL analysis. The sponge system is configured to determining blood loss volume contained within absorbent articles, such as surgical sponges. The user interface functions as a hub for to provide acute patient information to the attending medical personnel. The volume of blood loss may be displayed on the control panel and/or the user interface in real-time throughout the procedure. The volume of blood loss may also be displayed as a graphical plot over the time since the procedure was initiated. The user interface may trigger alarms, warnings, and all other critical information. The alarms or warnings may be based on thresholds or guidelines wirelessly pushed to the user interface.

BRIEF DESCRIPTION OF THE DRAWINGS

Advantages of the present disclosure will be readily appreciated as the same becomes better understood by reference to the following detailed description when considered in connection with the accompanying drawings.

FIG. 1 is a perspective view of a medical waste collection system with a manifold removably inserted into a receiver of the medical waste collection system.

FIG. 2 is a sectional view of the medical waste collection system of FIG. 1 with schematic representations of certain optional components of the medical waste collection system.

FIG. 3 is a perspective view of a manifold, and a representation of a fluid characterization module including a sensor assembly.

FIG. 4 is an exploded view of the manifold of FIG. 3 in which the manifold includes a head, a fluid director, a filter element, and a trunk.

FIG. 5 is a sectional perspective view of the manifold of FIG. 3 taken along section line 5-5.

FIG. 6 is a sectional elevation view of the manifold of FIG. 3 taken along section line 6-6.

FIG. 7 is a sectional elevation view of another manifold in which a first barrier of the fluid director is positioned proximal to an inlet fitting of the head.

FIG. 8 is an exploded view of another manifold in which the fluid director is positioned proximal to the filter element. The fluid characterization module may be disposed within the manifold.

FIG. 9 is a sectional elevation view of the manifold of FIG. 8 taken along section lines 9-9.

FIG. 10 is an exploded view of another manifold in which the flow of fluid is filtered by the filter element prior to encountering the fluid director.

FIG. 11 is a sectional elevation view of the manifold of FIG. 10 taken along section lines 11-11.

FIG. 12 is a perspective view of another manifold in which a projection defining a sump extends downwardly from the head of the manifold.

FIG. 13 is an exploded view of one variant of the manifold of FIG. 12 in which a second filter element and a straw are disposed within the sump.

FIG. 14 is a sectional elevation view of the manifold of FIG. 13 taken along section lines 14-14.

FIG. 15 is a sectional elevation view of another variant of the manifold of FIG. 12 in which the straw extends through the second filter element.

FIG. 16 is a perspective view of another manifold in which the fluid characterization module may be inserted through an accessory opening of the manifold.

FIG. 17 is perspective view of the fluid characterization module of FIG. 16.

**FIG.** 18 is a perspective view of the receiver of the medical waste collection system, and another manifold configured removably inserted into the receiver. The fluid characterization module may be coupled to the manifold.

FIG. 19 is a perspective view of the receiver of the medical waste collection system, and another manifold removably inserted into the receiver.

FIG. 20 is a sectional view of the receiver and manifold of FIG. 19 taken along section lines 20-20.

FIG. 21 is a rear respective view of a portion of the manifold of FIG. 19 in which a first leg of the manifold defines a detection window configured to be arranged near the sensor assembly of the receiver.

FIG. 22 is a perspective view of an inlet mechanism of the receiver. The sensory assembly is coupled to the inlet mechanism.

FIG. 23 is a schematic representation of electronic components of the fluid characterization module, and optionally, the medical waste collection system for quantifying blood loss.

FIG. 24 is a perspective view of another manifold in which a distal fluid director and a proximal fluid director may be actuated between a first fluid reservoir and a second fluid reservoir based on a fluid level within the first and second reservoirs.

FIG. 25 is a top plan view of the manifold of FIG. 24.

FIG. 26 is an implementation of the sensor assembly.

FIG. 27 is a graphical representation of molar extinction coefficient for a range of wavelengths of light for each of hemoglobin (Hb) and oxyhemoglobin (HbO$_2$).

FIG. 28 is an electrical schematic diagram including a microcontroller for adjusting the gain of the sensor assembly.

FIG. 29 is a graphical representation of light transmissivity ($U_{IN}$) and the gain ($U_{OUT}$) over time in which the gain is adjusted based on the light transmissivity relative to a predetermined threshold.

FIG. 30 is another graphical representation of light transmissivity ($U_{IN}$) and the gain ($U_{OUT}$) over time in which the gain is adjusted based on the light transmissivity relative to a predetermined threshold. The gain adjustment may account for hysteresis effects.

FIG. 31 is a representation of a blood management system including the medical waste collection system, a sponge system, and a user interface.

## DETAILED DESCRIPTION

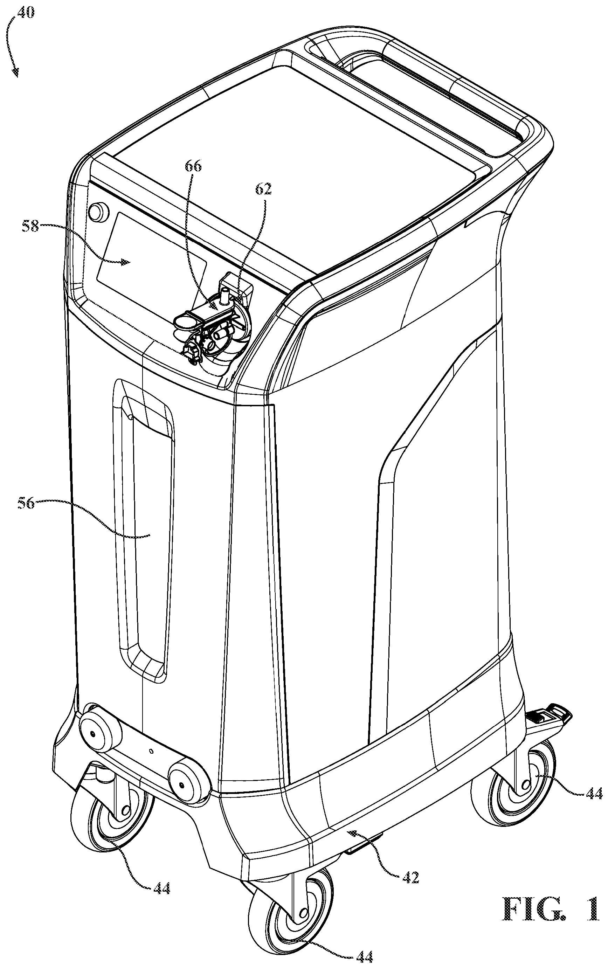

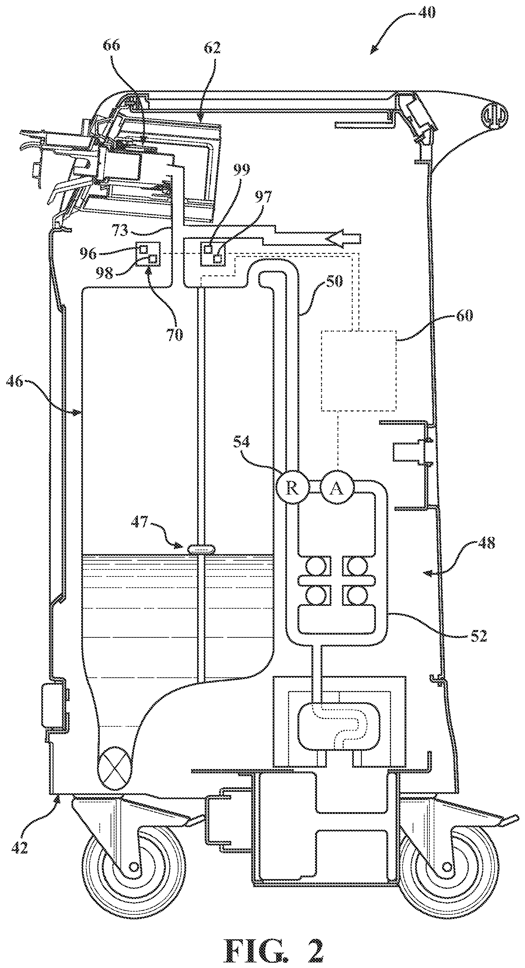

FIGS. 1 and 2 show a medical waste collection system 40 for collecting waste material generated during medical procedures, and more particularly surgical procedures. The waste material may include smoke, body tissues, and waste fluids such as bodily fluids and irrigation liquids. Often times, medical procedures require large amounts of saline and/or other irrigation liquids for irrigating an anatomical site. The medical waste collection system 40 collects the waste material and/or stores the waste material until it is necessary or desired to empty and dispose of the waste material. The medical waste collection system 40 may be transported to and operably coupled with a docking station through which the waste material is emptied. The docking station may include an off-load pump and a docking controller operatively coupled to the off-load pump. The docking station may otherwise assume any suitable form such as that disclosed in commonly-owned United States Patent No. 7,621,898, issued November 24, 2009.

The medical waste collection system 40 may include a chassis 42 and wheels 44 for moving the system 40 along a floor surface within a medical facility. The medical waste collection system 40 includes at least one waste container 46 defining a waste volume for collecting and storing the waste material. A vacuum source 48 may be supported on the chassis 42 and configured to draw suction on the waste container 46 through one or more internal lines 50. The vacuum source 48 may include a vacuum pump 52 and a vacuum regulator 54 (shown schematically in FIG. 2) supported on the chassis 42 and in fluid communication with the waste container 46. The vacuum regulator 54 is configured to regulate a level of the suction drawn by the vacuum pump 52 on the waste container 46. Suitable construction and operation of several subsystems of the medical waste collection system 40 are disclosed in commonly-owned United States Patent Publication No. 2005/0171495, published August 4, 2005, International Publication No. WO 2007/070570, published June 21, 2007, International Publication No. WO 2014/066337, published May 1, 2014, and International Publication No. 2017/15284, published June 29, 2017. In other configurations, the vacuum source 48 may be a separate unit that can be removably coupled to the medical waste collection system 40 to draw suction on the waste container 46. Suitable construction and operation of such an arrangement is disclosed in commonly-owned United States Patent No. 10,105,470, issued October 23, 2018.

A front of the chassis 42 may define a window 56 to permit a user to view the waste container 46. In implementations where the waste container 46 includes a transparent or translucent material, the user can see a level of waste material in the waste container 46 through the window 56. The medical waste collection system 40 may also include a light source (not shown) configured to illuminate the waste container 46 to assist the user in observing the level of waste material in the waste container 46. Particularly where the waste material includes bodily fluids such as blood and non-blood liquids, visualizing the contents of the waste container 46 may be particularly advantageous for qualitative assessment of the extent of blood loss. The qualitative assessment may be in addition to the quantitative blood loss (QBL) analysis to be described. For example, the user may visually monitor the color of the waste material through the window 56, and should the color become too reddish in color indicative of excessive blood loss, the user may elect to view a control panel 58 that displays the quantitative blood loss analysis.

The control panel 58 disposed on the chassis 42 is in communication with a controller 60 (shown schematically in FIG. 2) including a processor. The controller 60 is configured to generate signals to the vacuum regulator 52 to operate the vacuum regulator 52 to adjust the vacuum level in the waste container 46. In another configuration, the controller 60 is configured to generate signals to operate the vacuum source 38 to maintain or adjust the vacuum level in the waste container 46.

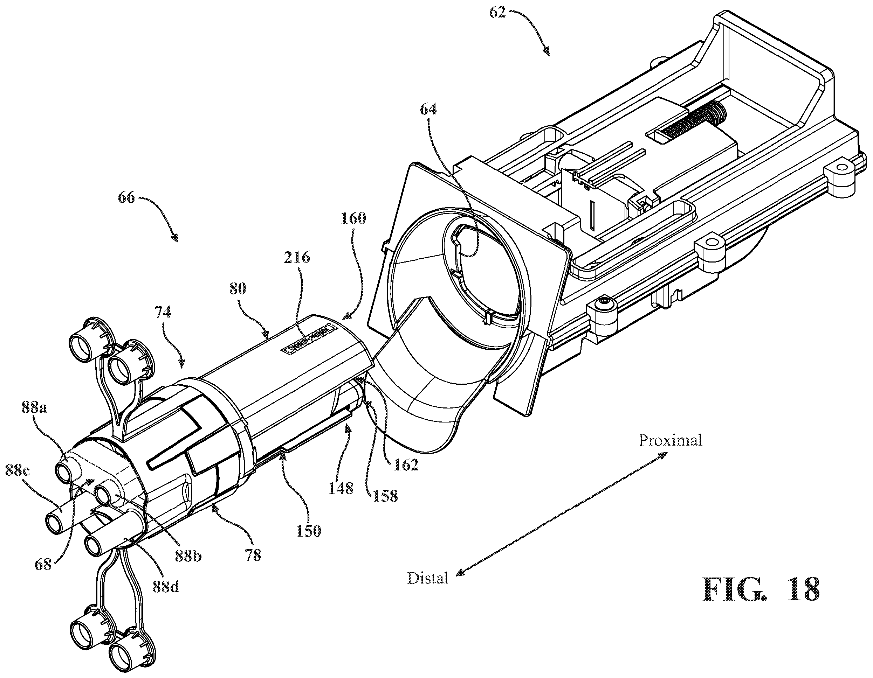

The medical waste collection system 40 includes at least one receiver 62 supported on the chassis 42. In a most general sense, the receiver 62 defines an opening 64 (see FIG. 18) sized to removably receive at least a portion of a manifold 66 to be described. FIG. 2 shows a single receiver, but two receivers associated with a respective one of the plural waste containers is contemplated. A suction path may be established from suction tube(s) to the waste container 46 through the manifold 66 removably inserted into the receiver 62. In other words, the vacuum generated by the vacuum source 38 is drawn on the suction tube(s), and the waste material at the surgical site is drawn through the manifold 66, through the receiver 62, and into the waste container 46. The manifold 66 includes features to be described that are configured to facilitate the QBL analysis of the inflow of the fluid. The manifold 66 may be a disposable component.

A fluid characterization module 68 is configured to facilitate quantifying a concentration of blood within the fluid being draw through the medical waste collection system 40 under the influence of suction. The quantification of the concentration of blood within the fluid may then facilitate the QBL analysis. The fluid characterization module 68 includes a sensor assembly 70, and further may include a module housing 72. In certain implementations, the fluid characterization module 68 is integral with or configured to be coupled with the manifold 66, and in other implementations, the fluid characterization module 68 is integrated with the medical waste collection system 40. FIG. 2 schematically represents an implementation in which the sensor assembly 70 of the fluid characterization module 68 is integrated with the medical waste collection system 40 by being coupled to or positioned adjacent an internal conduit 73 in fluid communication with the receiver 62. Other suitable locations of the fluid characterization module 68 are contemplated, such as locations adjacent or within the waste container 46 and/or the receiver 62. The fluid characterization module 68 is configured to generate a signal for determining a blood concentration within the fluid. In implementations in which the fluid characterization module 68 is integrated with the medical waste collection system 40, the conduit(s) 73 may be optically clear, which may discolor or otherwise foul over time. A cleaning line may be provided and is configured to direct water and/or detergent is directed through the cleaning line and the conduit(s) 73 to preserve its optical properties. On suitable cleaning system is disclosed in the aforementioned United States Patent No. 7,612,898.

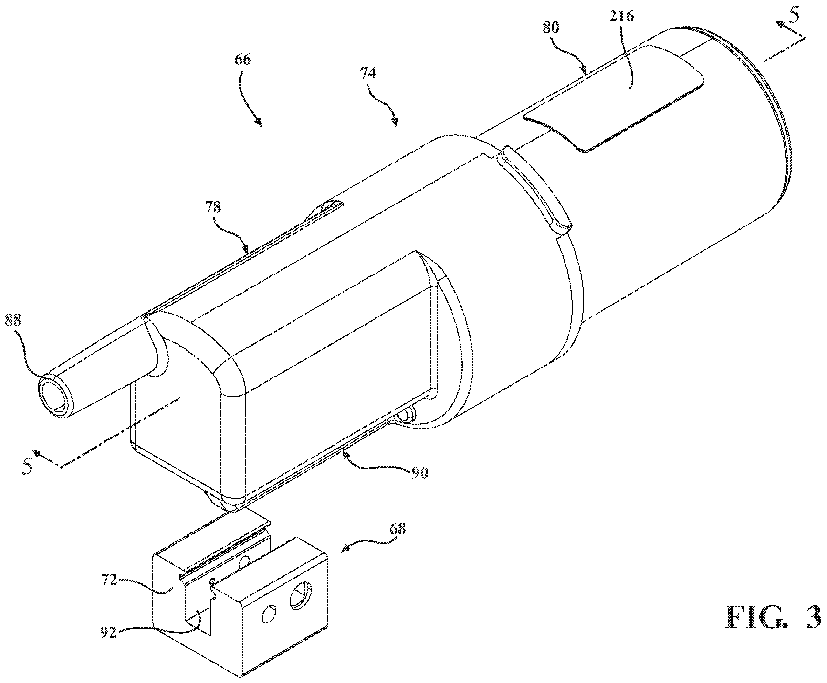

Referring now to FIGS. 3-6, an implementation of the manifold 66 is illustrated. The manifold 66 includes a housing 74 that defines a manifold volume 76. The manifold 66 may include a head 78 coupled to a trunk 80, or in alternative constructions the housing 74 of the manifold 66 may be of unitary or monolithic construction. The head 78 and the trunk 80 cooperate to define the manifold volume 76. The head 78 may include an inlet fitting 88 configured to removably receive at least one suction tube (not shown). The trunk 80 may define an outlet opening 82 in fluid communication with the manifold volume 76 and the inlet fitting 88. A seal 84 may be coupled to the housing 74 and sized to cover the outlet opening 82. A filter element 86 may be disposed within the manifold volume 76. The filter element 86, in a broadest sense, includes pores, apertures, or other structures configured to capture or collect the semisolid or solid waste material entrained within the fluid being drawn through the manifold 66 under the influence of suction. It should be appreciated that not all configurations of the manifold require use of the filter element, and the filter element may be disposed in a location separate from the manifold volume that is in fluid communication with the outlet opening 82 of the manifold 66.

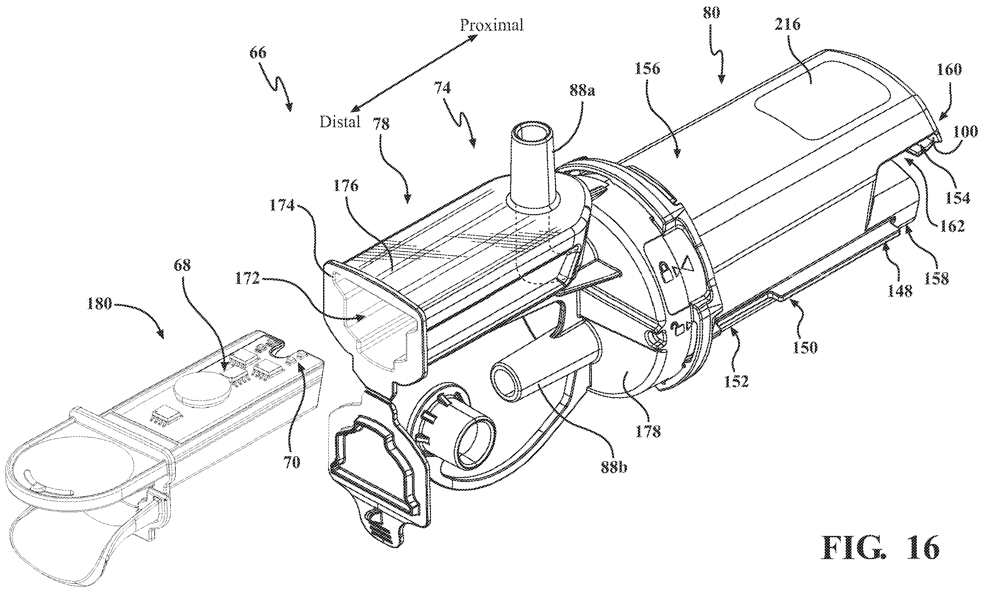

Certain implementations of the manifold 66 include the outlet opening 82 being offset from a longitudinal axis of the manifold 66 and configured to function as a valve driver of a rotatable valve of the medical waste collection system 40. More particularly, the trunk 80 may be generally cylindrical so as to be inserted into the receiver 62 and then rotated to establish fluid communication between the manifold volume 76 and the waste container 46 in a manner disclosed in commonly-owned United States Patent No. 7,615,037, issued November 10, 2009. In alternative implementations of the manifold 66, the trunk 80 includes features to be described that provide for insertion of the manifold 66 in a proximal direction and removal of the manifold from the receiver 62 the distal direction in a manner disclosed in commonly-owned International Publication No. WO2020/209898, published October 15, 2020. It is to be understood that the features of the manifold 66, in particular internal features related to facilitating the QBL analysis, may be included on either implementation of the trunk 80. In other words, features described with reference to FIGS. 3-15 may be included with the trunk 80 shown in FIGS. 16-21, and features described with reference to FIGS. 16-21 may be included with the trunk 80 shown in FIGS. 3-15.

The manifold 66 may include a projection 90. The projection 90 may function as a module coupler such that the module housing 72 of the fluid characterization module 68 may be operably coupled to the manifold 66. The projection 90 may disposed on the head 78 and extend longitudinally in the proximal-to-distal direction. The projection 90 may be elongate and have a width less than a length. The projection 90 may include a coupling feature configured to engage the module housing 72 of the fluid characterization module 68. For example, the coupling feature may be a rail be sized to be slidably positioned with a slot 92 defined by the module housing 72. Alternatively, the fluid characterization module 68 may be clipped or otherwise secured to the projection 90. It should be understood that any suitable structure of the housing 74 or other component of the manifold 66 may be configured to be removably coupled with the fluid characterization module 68.

At least a portion of the housing 74 of the manifold 66 is optically clear to define a detection window 94. The detection window 94 is configured to be positioned adjacent or between at least one emitter 96 and at least one sensor 98 of the sensor assembly 70. In manners to be further described, the emitter 96 and the sensor 98 of the sensor assembly 70 are configured to detect an optical characteristic of the fluid passing through the suction path. In certain implementations, the portion of the housing 74 defining the detection window 94 may be formed from transparent material, such as clear plastic. An entirety of the housing 74 may be formed from clear plastic with the portion of the housing 74 to be positioned between the emitter 96 and the sensor 98 constituting the detection window 94. In certain implementations, the housing 74 may be formed from semi-opaque or opaque material with cutouts sized to be fixedly joined with panels that are optically clear.

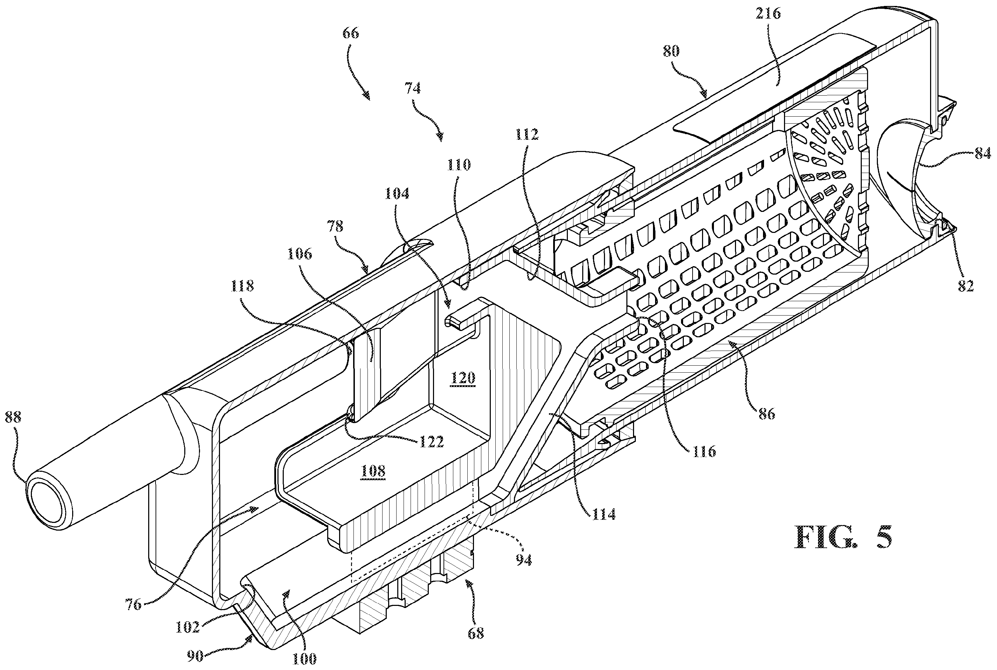

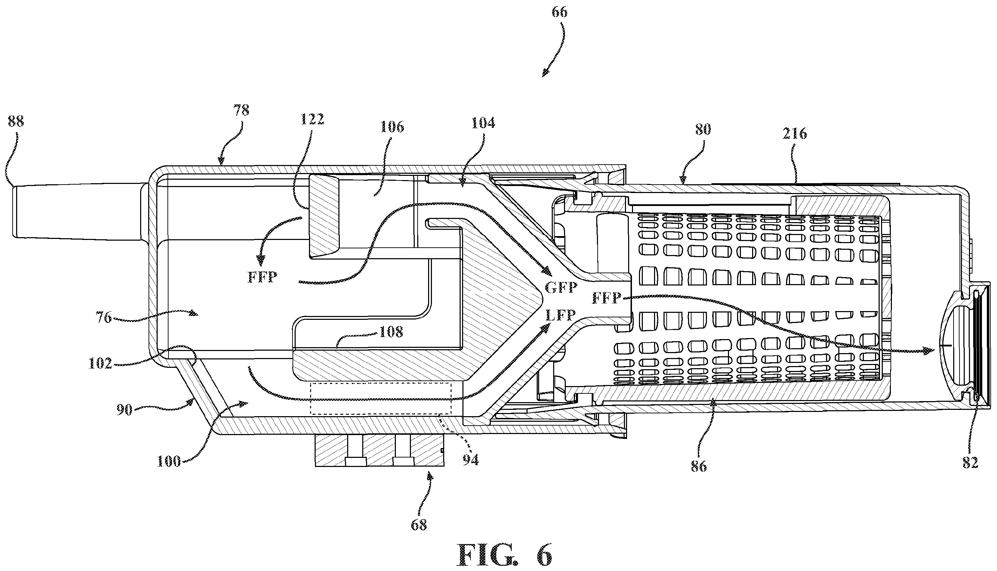

The implementation of FIGS. 5 and 6 shows the projection 90 defining the detection window 94. In such an arrangement, at least a portion of the projection 90 may be optically clear such that, with the projection 90 disposed in the slot 92 of the fluid characterization module 68, the sensor assembly 70 is positioned opposite the projection 90 to be in optical communication with the detection window 94.

The fluid characterization module 68 of FIG. 3 is shown as free-floating for illustrative purposes only. In one implementation, the module housing 72 is coupled to a dongle (not shown). The dongle may include data and power connections configured to be removably coupled with a complementary socket on the medical waste collection system 40. The data and power connections are established, and the module housing 72 defining the slot 92 may be slidably coupled along the projection 90, for example, from the front of the manifold 66. In another implementation, the fluid characterization module 68 is integrated with the receiver 62 such that the module housing 72 is not necessarily visible to the user. The manifold 66 is inserted into the receiver 62 such that the slot 92 is slidably coupled along the projection 90. It is understood that certain modifications may be necessary to the housing 74 of the manifold 66 to facilitate the removable coupling of the manifold 66 and the fluid characterization module 68 as the manifold 66 is inserted into the receiver 62 in the proximal direction.

As mentioned, the sensor assembly 70 includes the emitter(s) 96 and the sensor(s) 98. The emitters 96, 97 are configured to emit energy, and the sensors 98, 99 are configured to detect the emitted energy. An exemplary implementation is an optical sensor assembly that utilizes light energy with the emitters 96, 97 being light emitting diodes (LEDs) and the sensors 98, 99 being photodetectors. The emitters 96, 97 and the sensors 98, 99 are configured to be positioned opposite the detection window 94. Exemplary implementations include two emitters and two sensors and two emitters and four sensors, but it is understood that more or less of either may be provided. One of emitters (also referred to herein as the first emitter 96) may be positioned opposite the detection window 94 from two of the sensors, and the other one of the emitters (so referred to herein as the second emitter 97) is positioned opposite the detection window 94 from the other two of the sensors. The four sensors detect the emitted light, and more particularly the light after being transmitted or scattered through the fluid passing through the detection window 94. The detected intensity of the transmitted light or the scattered light may be indicative of the transmissivity, opacity, and/or other physical property of the fluid. In an exemplary arrangement, the first sensor 98 detects the transmitted light from the first emitter 96, the second sensor 99 detects the transmitted light from the second emitter 97, a third sensor (not shown) detects the scattered light from the first emitter 96, and a fourth sensor (not shown) detects the scattered light from the second emitter 97. The four measurements - two of transmitted light and two of scattered light - are values provided to the processor to execute an algorithm to determine the concentration of blood of the fluid passing through the detection window 94.

An alternative arrangement includes two sensors - the first sensor 98 and the second sensor 99 - with the first emitter 96 positioned opposite the detection window 94 of one of the sensors 98, and the second emitter 97 positioned opposite the detection window 94 of the other one of the sensors 99. The first sensor 98 detects the transmitted light from the first emitter 96 as well as the scattered light from the second emitter 97. The second sensor 99 detects the transmitted light from the second emitter 97 as well as the scattered light from the first emitter 96. Positioning the emitters 96, 97 on opposite sides of the detection window 94 limits or prevents crosstalk between the wavelengths of the light being emitted by the emitters 96, 97. In another implementation, the first emitter 96 and the second emitter 97 may be pulsed; *i.e.,* each illuminated in turn at a high frequency. The alternate pulsing of the first and second emitters 96, 97 may also limit or prevent crosstalk, and further reduce heat effects of the LEDs.

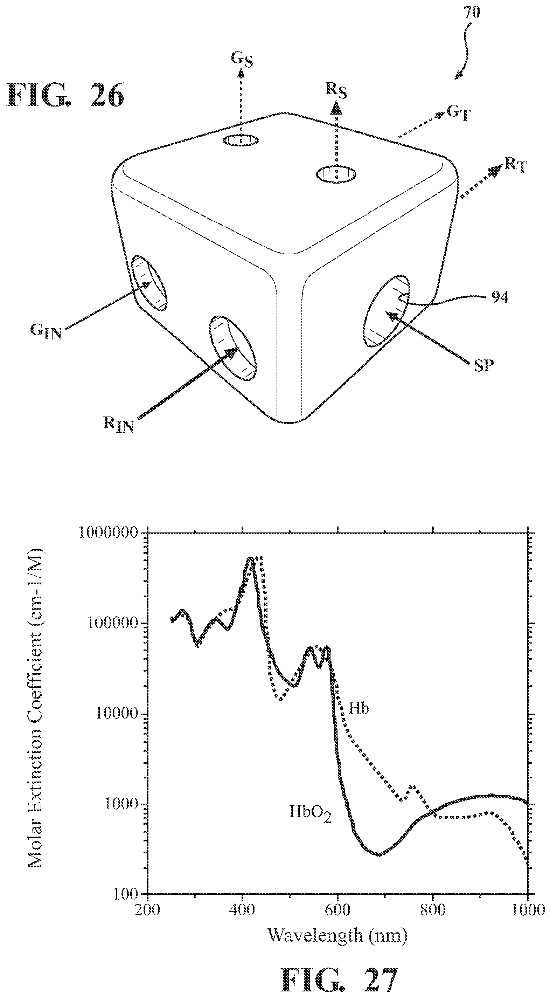

The implementation including two sensors may be particularly well suited for space-constrained applications with a schematic representation of the arrangement shown in FIG. 26. The module housing 72 of the fluid characterization module 68 defines at least one passage through which the fluid or liquid is directed, which is identified as the detection window 94. The arrows of FIG. 26 schematically represent the light being emitted and detected. Visible and infrared LEDs, for example, the first and second emitters 96, 97, are configured to direct visible light ($G_{IN}$) and infrared light ($R_{IN}$) into the module housing 72 and towards the fluid being drawn through the suction path (SP). The visible-light and infrared LEDs may be disposed within the openings of the module housing 72. As previously explained, some of the visible light may be transmitted through the fluid ($G_T$), and some of the visible light may be scattered ($G_S$) by the fluid. Likewise, some of the infrared light may be transmitted through the fluid ($R_T$), and some of the infrared light may be scattered ($R_S$) by the fluid. Photodetectors for example, the first and second sensors 98, 99, are configured to detect the transmitted visible light ($G_T$), the transmitted infrared light ($R_T$), the scattered visible light ($G_S$), and the scattered infrared light ($R_S$). The values provide the four values provided to the algorithm to determine the concentration of blood within the fluid. The photodetectors may be disposed within the openings of the module housing 72. There may be two or four photodetectors. The fluid characterization module 68 of FIG. 26 is a non-limiting design, and in particular the size and shape of the module housing 72 may be adapted to accommodate space constraints and/or the structure to which it is to be removably coupled. Likewise, the arrangement of the emitters 96, 97 and sensors 98, 99 may be in any suitable manner to obtain the requisite measurements of the transmitted light and the scattered light.

The first emitter 96 may be an infrared LED, and the second emitter 97 may be a visible-light LED. The infrared LED may be configured to emit light having a wavelength approximately in the range of 700 nanometers (nm) to 1000 nm, and more particularly within the range of 750 nm to 850 nm, and even more particularly within the range of 770 nm to 810 nm. The visible-light LED may be configured to emit light having a wavelength approximately in the range of 400 nm to 600 nm, and more particularly within the range of 550 nm to 600 nm, and even more particularly within the range of 570 nm to 580 nm. The visible-light LED may be a green LED. FIG. 27 is a graphical representation of molar extinction coefficient across a range of wavelengths for each of hemoglobin (Hb) and oxyhemoglobin (HbO2). Hemoglobin is the predominant protein in red blood cells, and oxyhemoglobin is oxygen-loaded form of hemoglobin that is bright red in color. The redness of the blood, which is dictated by the oxyhemoglobin, affects the transmissivity and scatter of the light being directed through the blood. The light transmissivity may be the characteristic of the fluid detected by the fluid characterization module 68. It has been determined that green light and infrared light may be optimal for determining the concentration of blood within the fluid. A measurement of the infrared light absorbed by the fluid is determined by a reduction in transmitted light due to the presence of the blood within the fluid. In one implementation, a ratio of this absorbance of the infrared light to scattered light from the visible light is calculated and used to quantify the concentration of blood in the fluid.

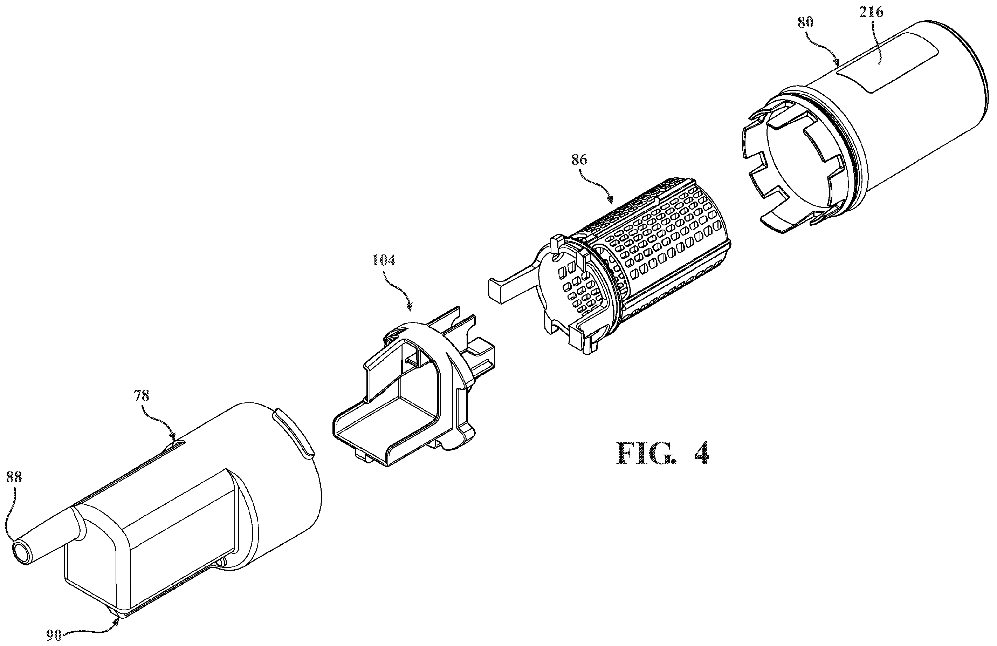

It has been observed that the waste material being suctioned through the manifold 66 may include a mixture of gas and liquid, for example, air and blood, respectively. The gas-liquid mixture may be due to gas and liquid being suctioned into the suction tube coupled to the inlet fitting 88, and/or gas bubbles generated by collisions and turbulence within internal geometries of the manifold 66. The presence of gas within the liquid may affect its optical properties, and therefore may undesirably compromise the accuracy of the measurements of the transmitted light and/or the scattered light. With continued reference to FIGS. 4-6, the projection 92 may define a sump 100, and the sump 100 is configured to facilitate the gas in the fluid separating from the liquid in the fluid prior to the fluid characterization module 68 measuring the transmitted light and the scattered light. As best shown in FIGS. 5 and 6, the sump 100 may be positioned below the manifold volume 76. For convention, a liquid inlet 102 may define a boundary between the manifold volume 76 and the sump 100, but the sump 100 may also be considered a subvolume of the manifold volume 76. Owing to a relative size of the liquid inlet 102, the fluid accumulates within the sump 100, and further may accumulate within the manifold volume 76. The accumulation of the fluid within the sump 100 on provides a brief period of time during which the gas may separate from the liquid, for example, bubbles separating from the blood and other liquid(s) under principles of bubble dynamics.

The manifold 66 may further a fluid director 104 disposed within the housing 74. The fluid director 104 includes various geometries configured to provide a torturous path to the fluid within the manifold 66. Among other advantages, the tortuous path may limit turbulent flow within the manifold 66, facilitate the separation of the air from the liquid within the fluid, and provide for filtering of the fluid upstream of the sensor assembly 70. With continued reference to FIGS. 4-6, the fluid director 104 is disposed within the manifold volume 76, and may at least partially disposed within the head 78. The geometries to be described may cooperate with internal geometries of the housing 74 to define a fluid flow path (FFP)(*i.e.*, including liquid and gas), a liquid flow path (LFP), and a gas flow path (GFP). With gas being less dense than liquid, the gas flow path may be positioned near an upper aspect of the manifold 66, whereas the liquid flow path may be positioned near a lower aspect of the manifold 66. In the illustrated implementation, the liquid flow path is at least partially defined by the sump 100 that includes the detection window 94.

The fluid director 104 may include a first barrier 106 and a second barrier 108, and define a gas inlet 110, a gas channel 112, a liquid channel 114, and a fluid outlet 116. Further, the second barrier 108 and the housing 74 of the manifold 66 may cooperate to define the liquid inlet 102 between the manifold volume 76 and the sump 100. The first barrier 106 is configured to impart the tortuous path to the fluid entering the manifold 66 through the inlet fitting 88, and more particularly through a proximal end 118 of the inlet fitting 88. The tortuous path together with the size of the liquid inlet 102 may result in at least some accumulation of fluid within the manifold volume 76. As mentioned, the accumulation provides a brief period of time during which the gas may separate from the liquid. The separated gas occupies a portion of the manifold volume 76 above the liquid and is suctioned through the gas inlet 110 from the vacuum provided by the medical waste collection system 40. The separated gas further is suctioned through the gas channel 112 and through the fluid outlet 116 to pass through the filter element 86 and the outlet opening 82. The separated liquid may be simultaneously suctioned through the liquid inlet 102 from the vacuum provided by the medical waste collection system 40. The separated liquid may be further is suctioned through the sump 100 including the detection window 94, the liquid channel 114, and the fluid outlet 116. The liquid passing the detection window 94 to be measured by the fluid characterization module 68 contains less, little or no gas, and therefore accuracy of the measurements from the fluid characterization module 68 may advantageously be preserved. For example, the relative absence of gas bubbles eliminates optical interference from the refractivity of the surfaces of the gas bubbles.

As best shown in FIGS. 5 and 6, the liquid flow path and the gas flow path may be joined prior to the fluid outlet 116. In particular, the liquid channel 114 and the gas channel 112 merge prior to the fluid outlet 116. As the liquid has already been measured by the fluid characterization module 68 upstream of the joining of the liquid and gas flow paths, it may not be necessary to maintain their separation as the fluid is suctioned through the remainder of the manifold 66. Thus, the detection window 94 may be positioned between the liquid inlet 102 and the liquid channel 114 and fluidly separated from the gas channel 112 (based on direction of suction through the manifold 66).

To facilitate the tortuous path and accumulation of fluid within the manifold volume 76, the first barrier 106 is positioned adjacent the proximal end 118 of the inlet fitting 88 within the head 78. More particularly, the first barrier 106 may extend distally from a rear barrier 120 to a distal edge 122 that is positioned distal to the proximal end 118 of the inlet fitting 88. In other words, the first barrier 106 and the inlet fitting 88 may "overlap" in the elevation view of FIG. 6. Further, the liquid inlet 102 may be positioned distal to the proximal end 118 of the inlet fitting 88, and the first barrier 106 may further be positioned adjacent the gas inlet 110. In such an arrangement, the first barrier 106 prevents a direct path from the proximal end 118 of the inlet fitting 88 to the gas inlet 110, effectively requiring the fluid flow path double-back on itself at least once for either of the gas inlet 110 or the liquid inlet 102 to be accessible to the inflow of the fluid. The inlet fitting 88 may be laterally offset towards the wall of the housing 74 to achieve the overlapping positioning relative to the first barrier 106. The first barrier 106 (and/or the housing 74) may be contoured to promote less turbulent flow, which may further promote separation of the gas from the liquid. The fluid flow path may accumulate in the manifold volume 76 with portions of the accumulated liquid being suctioned from below and through the liquid inlet 102 as previously described, and with portions of the gas separated from the liquid being suctioned from above and through the liquid inlet 102 as previously described. The dimensions of the liquid inlet 102 in view of anticipated inflow rates entering the manifold volume 76 may ensure the desired amount of accumulation, for example, to provide adequate opportunity for the gas to separate from the liquid.

In instances where the fluid is mostly gas with little liquid, the manifold volume 76 empties efficiently through the sump 100 as previously described. In instances where the fluid is mostly fluid, the accumulation of the liquid within the manifold volume 76 may reach the gas inlet 110. The gas inlet 110 may function as an overflow opening, after which both gas and liquid may be suctioned through the gas channel 112 and the fluid outlet 116 of the fluid director 104. Any blockage or loss of suction is prevented with a continuous flow of the liquid being suctioned through the liquid flow path and measured within the detection window 94.

The relative geometries of the gas inlet 110, the manifold volume 76, and the liquid channel 114 may be designed such that potential blockage is minimized and inflow through the inlet fitting 88 is maximized. For example, the outflow rates of the liquid through the liquid flow path may be based on a dimension of the liquid channel 114, and perhaps more importantly a dimension of the sump 100 defined by the projection 90. As mentioned, the detection window 94 may be a portion of the projection 90 that is optically clear, and in certain implementations, an entirety of the head 78 may be optically clear. Owing to the relatively high light absorptivity of blood, particularly at higher concentrations, it is beneficial for the emitters 96, 97 and the sensors 98, 99 - positioned opposite the projection 90 - to be sufficiently close for improved measurement accuracy. Therefore, in certain implementations, a width of the projection 90, and thus the detection window 94 is no greater than 19.05 mm (three-quarters of an inch), and more particularly approximately 12.7 mm (one-half inch). The width of the projection 90 may influence the flow rates of the liquid through the detection window 94, and therefore influence the outflow rates of the liquid through the liquid flow path. Further, limiting the width of the projection 90 may correspondingly limit the required operating ranges of the emitters 96, 97 and the sensors 98, 99, thereby improving accuracy with lower cost electronic components. In addition to quantifying blood loss, implementations with the fluid director 104 may also be utilized with other applications of continuous flow measurement may benefit from removing gas from the liquid, such as intravenous pumps and arthroscopies.

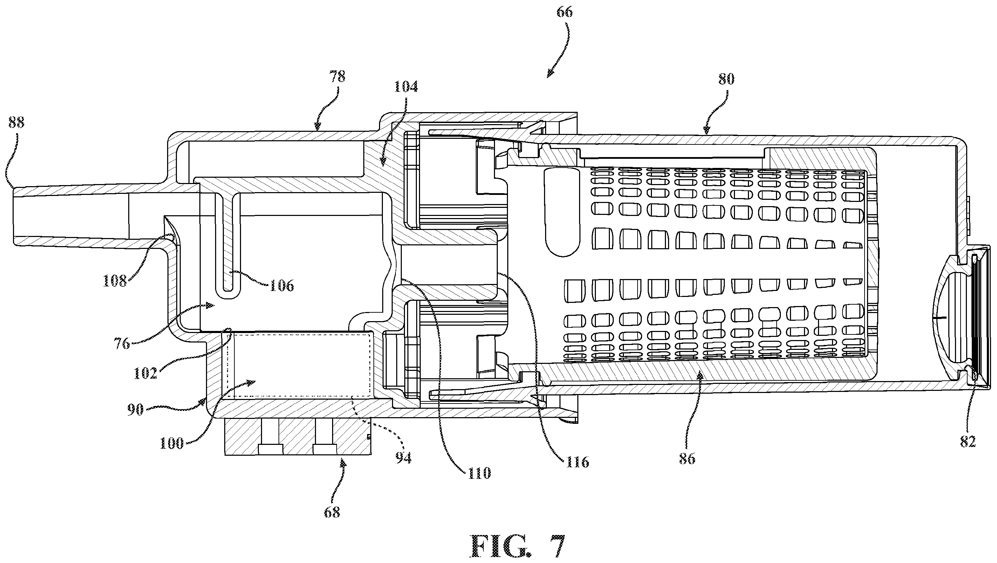

An alternative implementation of the fluid director 104 is shown in FIG. 7 in which there may not be a separate liquid channel defined by the fluid director 104. Rather, the first barrier 106 is positioned proximal to the proximal end 118 of the inlet fitting 88 to encounter in the incoming fluid and direct it in less turbulent manner to the manifold volume 76 and the sump 100. The depositing of incoming fluid into the sump 100 may agitate the accumulated fluid, thereby facilitating homogeneity of the fluid collected in the sump 100 that is being measured by the fluid characterization module 68. The inlet 110 may initially provide the gas channel, yet once sufficient fluid accumulates within the sump 100 and the manifold volume 76, the inlet 110 functions as an overflow opening, after which both gas and liquid may be suctioned through the fluid outlet 116 of the fluid director 104.

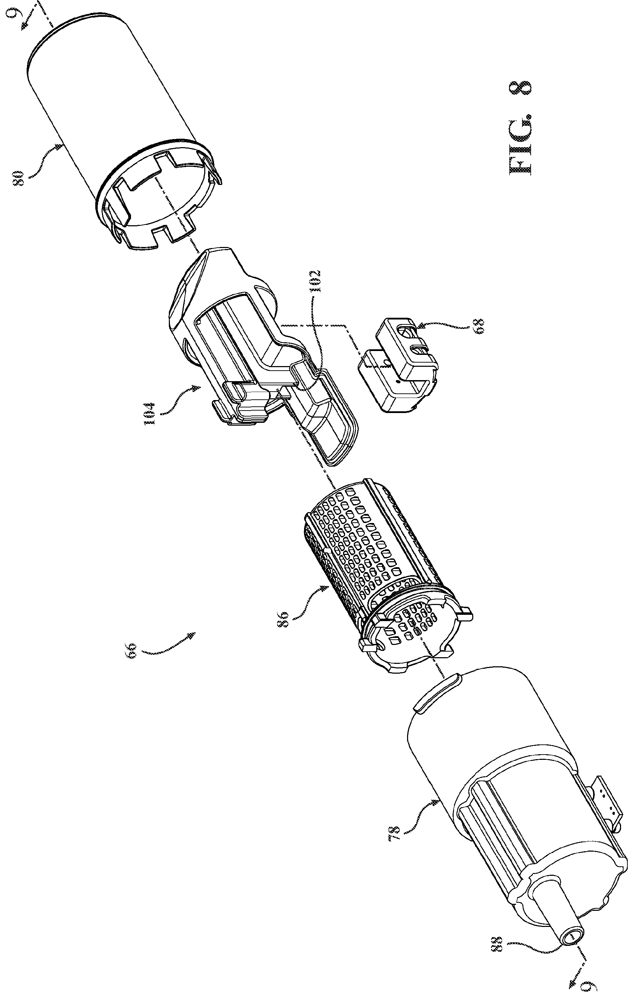

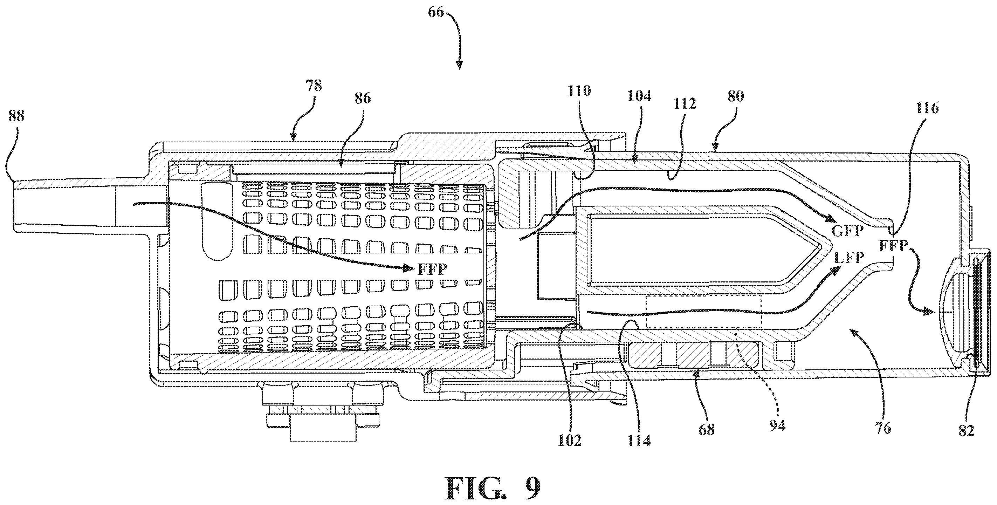

Referring now to FIGS. 8 and 9, another implementation of the manifold 66 is shown in which the fluid director 104 is positioned proximal to the filter element 86. In other words, the fluid director 104 is positioned closer to the outlet opening 82 than the filter element 86. In such an arrangement, the fluid entering the manifold volume 76 is filtered by the filter element 86 prior to encountering the detection window 94. Consequently, any tissue or semisolid matter that may affect the optical characteristics of the fluid is removed from the fluid. In other respects, the fluid director 104 may be similar in function the implementation previously described with like numerals identifying like components. In particular, FIG. 9 shows the fluid director 104 including the liquid inlet 102, the gas inlet 110, and the fluid outlet 116. The fluid director 104 defines the gas channel 112 between the gas inlet 110 and the fluid outlet 116, and further defines the liquid channel 114 between the liquid inlet 102 and the fluid outlet 116.

In the present implementation, the fluid director 104 defines the detection window 94. More particularly, the liquid channel 114 may define the detection window 94, and therefore at least a portion of the fluid director 104 is optically clear. In the present implementation, the fluid characterization module 68 is disposed within the manifold 66. FIG. 9 generally shows the fluid characterization module 68 positioned within the trunk 80 with the liquid channel 114 disposed adjacent or between the sensor assembly 70 of the fluid characterization module 68. The implementation may necessitate the fluid characterization module 68 including a communications module (not identified) that wirelessly transmits signals to the medical waste collection system 40 such that the controller or processor 60 utilizes the signals in real-time to determine the blood concentration and resultingly perform the QBL analysis. Alternatively, modifications to the trunk 80 are contemplated such that the fluid characterization module 68 is positioned external to the manifold 66 and in optical communication with the fluid director 104 through the trunk 80, a portion of which may also be optically clear. With the fluid characterization module 68 engaging the liquid channel 114 of the fluid director 104, it is appreciated that the present implementation does not include the projection defining the sump while still realizing adequate separation of the gas and liquid in the fluid prior to measuring the optical characteristics of the fluid.

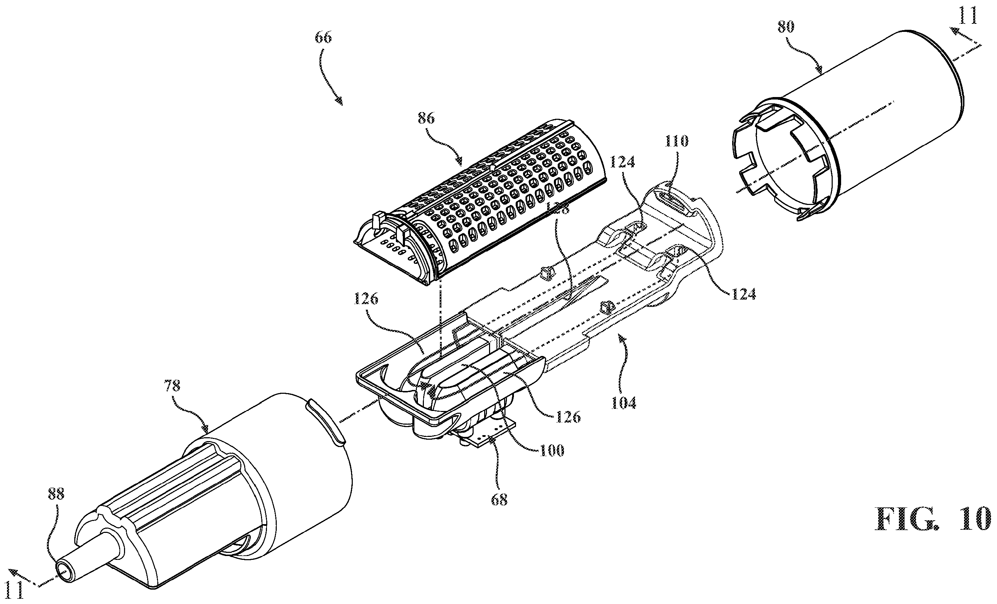

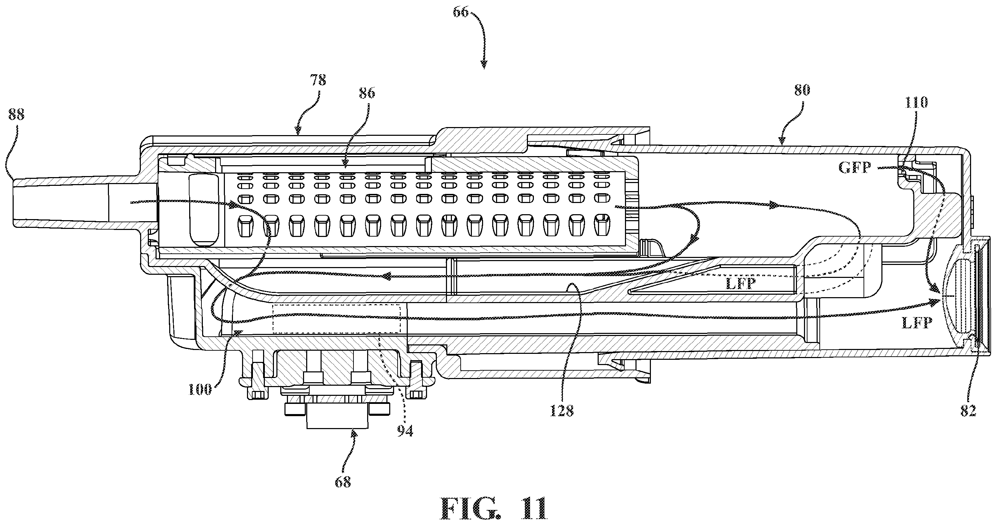

FIGS. 10 and 11 show another implementation of the manifold 66 in which the fluid is filtered by the filtering element 86 prior to encountering the detection window 94. Whereas the previous implementation included the fluid characterization module 68 disposed within the trunk 80 of the manifold 66 to account for the distal positioning of the filter element 86 relative to the detection window 94, the fluid director 104 of the present implementation redirects the fluid distally towards the sump 100. As a result, the fluid characterization module 68 may be coupled to an exterior of the head 78 of the manifold 66 (and possibly external to the receiver 62) despite the inflow of the fluid first passing through the filter element 86. The fluid director 104 includes at least one redirecting aperture 124 in fluid communication with a lateral channel 126 extending longitudinally within the manifold 66. The fluid director 104 further defines the sump 100 in communication with the lateral channels 126, and further in communication with the outlet opening 82. Still further, the fluid director 104 may define a central channel 128 in communication with the sump 100, and the gas inlet 110 near a proximal end of the manifold 66. Ridges may separate the sump 100 from the lateral channels 126 with the ridges sized to receive the fluid characterization module 68. The sump 100 is positioned between the sensor assembly 70. In order to accommodate the lateral channels 126, the filter element 86 may be non-cylindrical and positioned in a stacked arrangement with the fluid director 104. For example, the filter element 86 may be hemicylindrical with a flat surface arranged to be supported atop an upper surface of the fluid director 104.

With reference to the arrows annotated in FIGS. 9 and 10, the fluid enters the manifold 66 through the inlet fitting 88, after which it is filtered by the filter element 86. Some of the fluid may pass through pores in a base of the filter element 86 and into the sump 100, and some of the fluid passes through a proximal end of the filter element 86 and into the manifold volume 76. The liquid within the fluid may be drawn through the central channel 128 towards the sump 100 while the gas separates to be drawn through the gas inlet 110 towards the outlet opening 82. Some of the fluid is drawn through the redirecting apertures 124 to be distally directed along the lateral channels 126. Near a distal end of the manifold 66, the fluid is again redirected from the distal direction to the proximal direction to enter the sump 100. The optical characteristics of the fluid passing through the sump 100 is measured by the sensor assembly 70 of the fluid characterization module 68, after which it is drawn under suction towards the outlet opening 82.

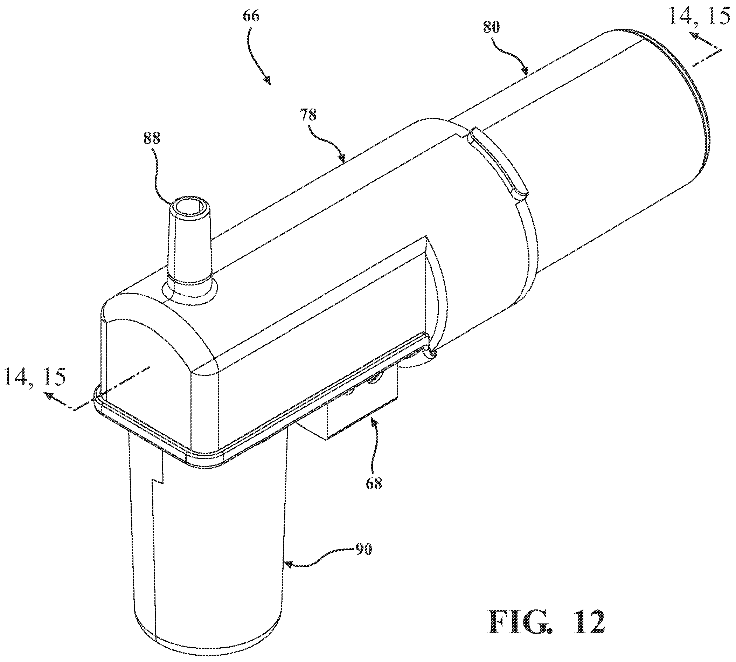

In certain implementations, a second filter element 130 may be provided. Referring now to FIGS. 12-16, the projection 90 of the manifold 66 may be shaped and sized to accommodate the second filter element 130. The illustrated implementation shows the projection 90 being cylindrical and extending downwardly from the head 78 of the manifold 66. Owing to the size and shape of the projection 90, the sump 100 of the present implementation is configured to accommodate larger amounts of the fluid, for example, to permit ample separation of the gas and the liquid within the fluid. The fluid characterization module 68 is positioned proximal to the sump 100, and therefore the size of the projection 90 may not be constrained by the technical limits of sensor assembly 70.

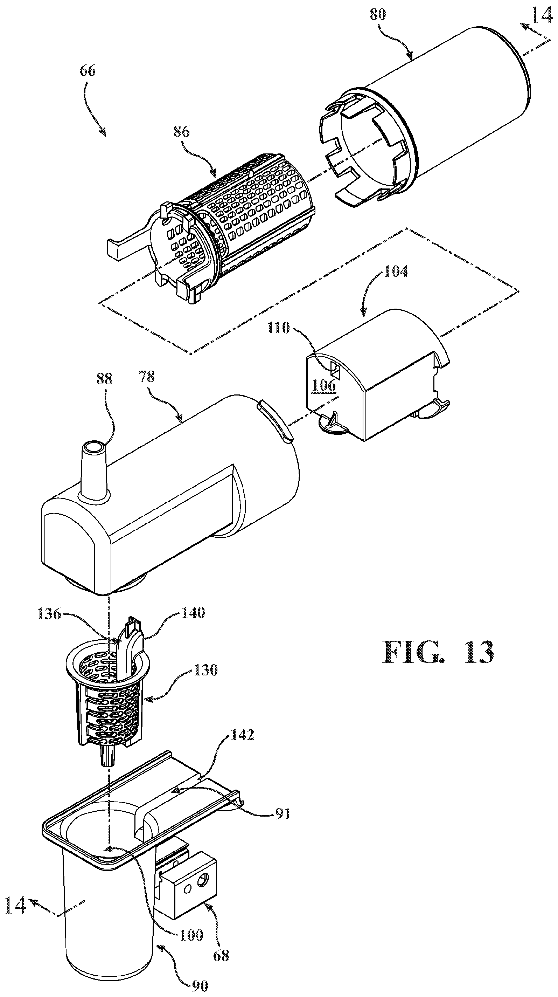

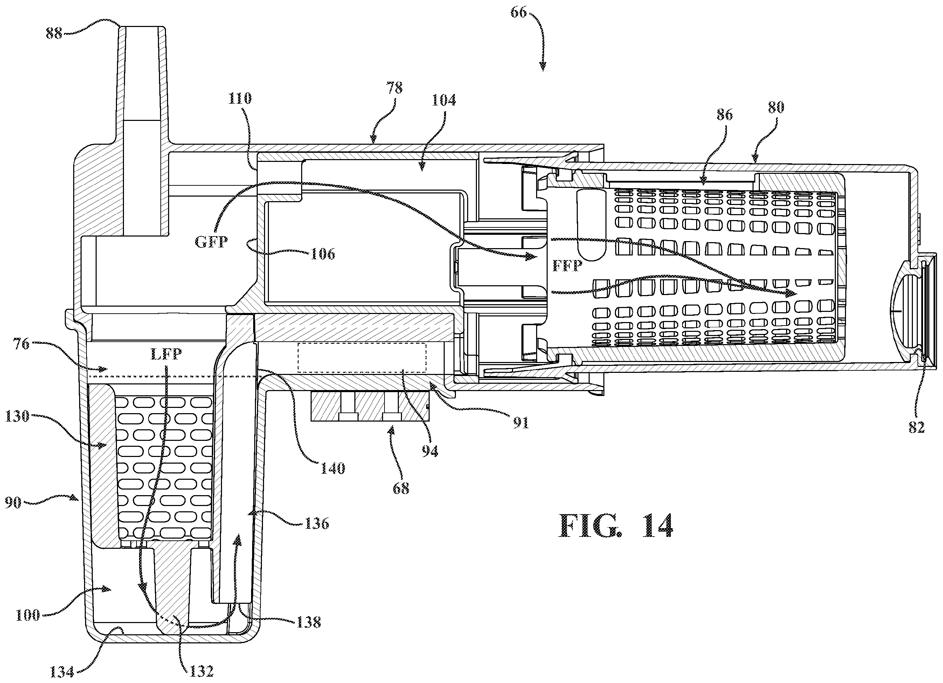

The second filter element 130 is disposed within the sump 100. In a first variant shown in FIGS. 13 and 14, the second filter element 130 includes a spacer 132 configured to abut a base 134 of the projection 90 and provide clearance between the second filter element 130 and the base 134. A straw 136 is at least partially disposed within the sump 100. The straw 136 includes a first end 138 disposed within the sump 100, and a second end 140 disposed within the manifold volume 76 (a dashed line demarcates a boundary between the sump 100 and the manifold volume 76). The fluid director 104 includes the first barrier 106 that defines the gas inlet 110. The first barrier 106 requires the inflow of fluid through the inlet fitting 88 be directed into the sump 100 under the influence of gravity. Within the sump 100, the gas and the liquid within the fluid may separate in the manner previously described. The gas within the manifold volume 76 is suctioned through the gas inlet 110 and towards the outlet opening 82. The liquid accumulates within the sump 100.

The balance of the vacuum provided by the system 40 is drawn through the straw 136. In other words, the vacuum on the second end 140 of the straw 136 draws the liquid from the sump 100 through the first end 138 of the straw 136 against the force of gravity. The liquid is drawn through the straw 136, and further through the detection window 94 defined by a second projection 91. For convention, the second projection 91 may be akin to the certain implementations of the (first) projection 90 previously described by being elongate and configured to be positioned within the slot 92 of the module housing 72. As best shown in FIG. 13, the second projection 91 may define a channel 142 in fluid communication with the sump 100 through the straw 136. The optical characteristics of the liquid passing the detection window 94 is measured by the fluid characterization module 68.

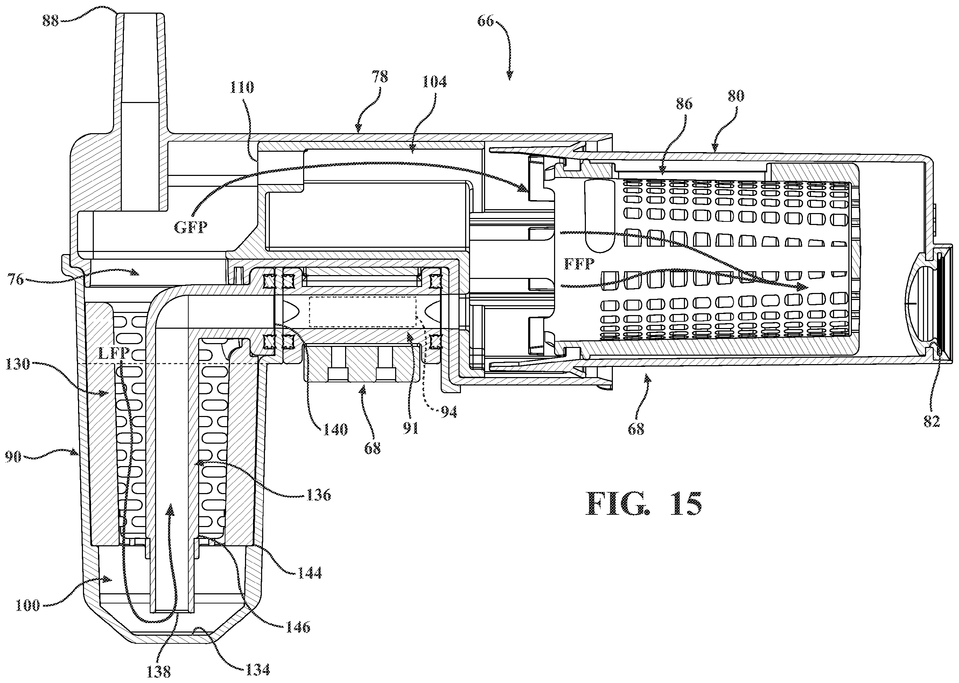

In another variant, the straw 136 may extend through the second filter element 130. With reference to FIG. 15, the projection 90 may be tapered and/or define a step 144, and the second filter element 130 is supported on the step 144 to provide clearance above the base 134 of the projection 90. A base of the second filter element 130 defines an opening, and the straw 136 includes a step 146 supported within the opening. With the taper to the base 134 of the projection 90 and the first end 138 of the straw 136 being centrally located within the sump 100, improved performance through the straw 136 may be realized. The liquid is drawn through the straw 136 and through the detection window 94 of the second projection 91.

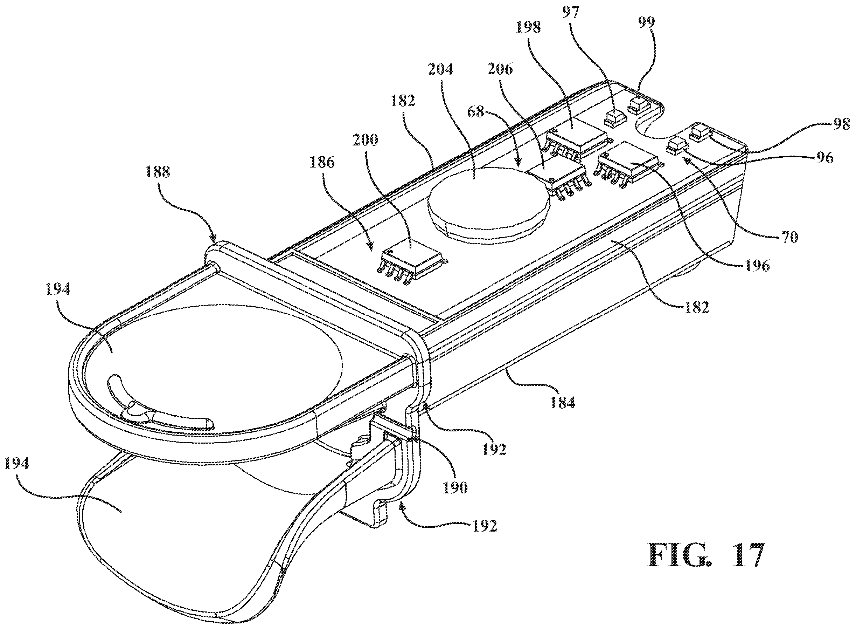

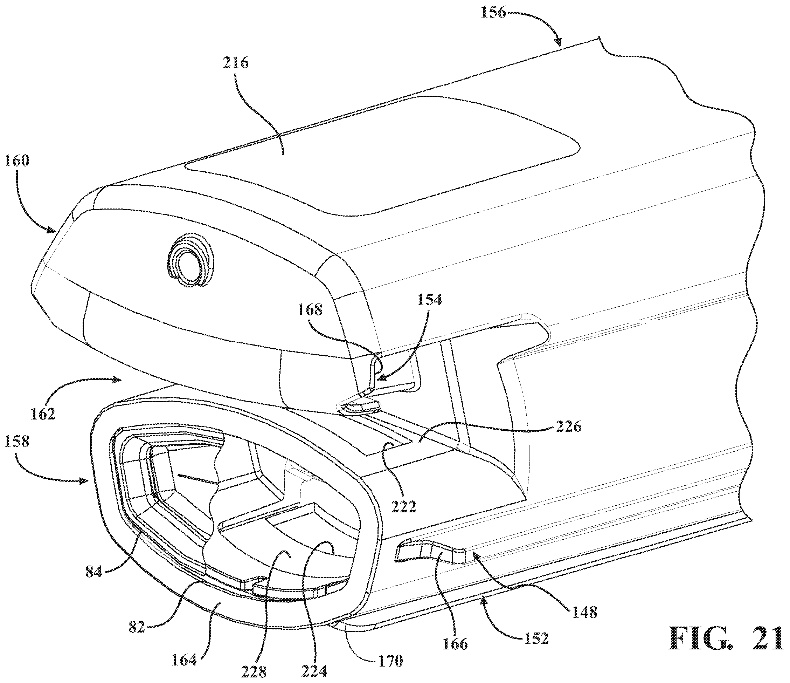

Referring now to FIGS. 16 and 17, another implementation of the manifold 66 is shown in which the fluid characterization module 68 may be removably inserted into a portion of the housing 74 of the manifold 66. First describing features of the trunk 80 configured to engage complementary features of the receiver 62 of the medical waste collection system 40, the manifold 66 includes an arm 148, a lock element 150, a spine 152, and/or a catch 154. For convention the directional references (e.g., proximal, distal, upper, lower, above, below, etc.) are made with the manifold 66 in the orientation shown in FIG. 16 in which the manifold 66 is inserted into the receiver 62. The housing 74 may include a body portion 156, a first leg 158, and/or a second leg 160. The first leg 158 and/or the second leg 160 may extend from the body portion 156, and more particularly one or both of the first and second legs 158, 160 may extend proximally from the body portion 156. The first leg 158 may be positioned below the second leg 160 when the manifold 66 is oriented for insertion into the receiver 62. The first and second legs 158, 160 may be spaced apart from one another by a void 162, as best shown in the rear perspective view of FIG. 21. It is understood that potentially trivial changes in the illustrated geometries may be included without deviating from the above conventions. The housing 74 may include a rim 164 defining the outlet opening 82. The rim 164 may be disposed on the first leg 158, and more particularly at or near a proximal end of the first leg 158. In one convention, the rim 164 may be considered a proximally-directed surface at the proximal end of the first leg 158. The rim 164 may include a width greater or larger than a height such that the outlet opening 82 is non-circular. The rim 164 may be configured to be coupled with the seal 84.

The manifold 66 includes the arm 148 extending outwardly from the housing 74. A pair of arms 148 are referenced, but it is appreciated that a singular arm may be provided. FIGS. 16 and 21 show the arms 148 as elongate rib-like structures in the proximal-to-distal direction and including a width greater or larger than a thickness. The arms 148 may be sized and shaped to movably be inserted arm slots defining the opening 64 of the receiver 62 (see FIG. 18). It should be appreciated that not all configurations of the manifold 66 require use of the arms 148, and manifold designs that do not include arms are contemplated. The arms 148 each include a proximally-directed surface 166 configured to engage the receiver 62 during insertion of the manifold 66 into the receiver 62 to facilitate moving the receiver 62, and components thereof, between certain operative positions. The proximally-directed surfaces 166 of the arms 148 may be positioned distal to the rim 164.

The manifold 66 includes the catch 154 with a pair of catches 154 to be described. It should be appreciated that a singular catch may be provided, and manifold designs that do not include the catch(es) are contemplated. The catches 154 may be disposed on the second leg 160. Each of the catches 154 includes a distally-directed surface 168 configured to be engaged by claws of the receiver 62 during insertion and removal of the manifold 66 into the receiver 62 to facilitate moving the receiver 62. The distally-directed surfaces 168 of the catches 154 may be positioned proximal to the rim 164, and positioned proximal to the proximally-directed surfaces 166 of the arms 148. The rim 164 and at least one of the catches 154 may be spaced apart from one another by the void 162. More particularly, the rim 164 on the first leg 158 may be spaced apart from the catches 154 on the second leg 160 by the void 162. In other words, the rim 164 may be on a first or lower side of the void 162, and the catches 154 may be on a second or upper side of the void 162 opposite the first or upper side. Further, the rim 164 is positioned below the catches 154 when the manifold 66 is oriented for insertion into the receiver 62.

The manifold 66 may include the spine 152 extending outwardly from the housing 74. The spine 152 may be an elongate structure in the proximal-to-distal direction and including a width greater or larger than a thickness. The spine 152 may extend outwardly from at least one of the body portion 156 and/or the first leg 158. Further, the spine 152 may extend downwardly from the bottom wall of the trunk 80. The spine 152 includes a proximally-directed surface 170 configured to engage a sled lock assembly of the receiver 62 during insertion and removal of the manifold 66 into the receiver 62 to facilitate moving the receiver 62, and components thereof, between the operative positions. The proximally-directed surface 170 of the spine 152 may be positioned distal to the rim 164, positioned distal to the distally-directed surfaces 168 of the catches 154, and positioned distal to the proximally-directed surfaces 166 of the arms 148. In certain implementations, the proximally-directed surface 170 is inclined towards the housing 74 in the proximal direction to define a proximal end of the spine 152. The incline may be a ramped surface.

The manifold 66 includes the lock element 150 extending outwardly from the housing 74. A pair of lock elements 150 are referenced throughout the present disclosure, but it is appreciated that a singular lock element may be provided, and manifold designs that do not include a lock element are contemplated. FIG. 16 show each of the lock elements 150 as sharing the elongate structure as a respective one of the arms 148. In particular, the lock elements 150 each may include a distally-directed surface at a distal end of the elongate structure opposite the proximally-directed surface 166 of the arms 148. The lock elements 150 may extend outwardly from at least one of the body portion 156 and the first leg 158. The distally-directed surfaces are configured to engage a locking assembly of the receiver 62 after insertion of the manifold 66 into the receiver 62 to selectively prevent distal movement of the manifold 66 relative to the receiver 62. The distally-directed surfaces of the lock elements 150 may be positioned distal to the rim 164, positioned distal to the distally-directed surfaces 168 of the catches 154, positioned distal to the proximally-directed surfaces 166 of the arms 148, and positioned distal to the proximally-directed surface 170 of the spine 152. The relative positioning in the proximal-to-distal direction of each of the rim 164, the proximally-directed surfaces 166 of the arms 148, the distally-directed surfaces 168 of the catches 154, the proximally-directed surface 170 of the spine 152, and/or the distally-directed surfaces of the lock elements 150 are tuned to facilitate precise operative timing of complementary components of the receiver 62 as the manifold 66 is inserted within the receiver 62.

The head 78 is positioned distal to the trunk 80 when the manifold 66 is oriented for insertion into the opening 64 of the receiver 62. The head 78 may include the first inlet fitting 88a, and a second inlet fitting 88b. More particularly, the head 78 may define an accessory sleeve 172 extending from an accessory opening 174, and the first inlet fitting 88a may extend upwardly from an upper barrier 176 of the accessory sleeve 172. The second inlet fitting 88b extends distally from a cap faceplate 178 and defines a second inlet bore (also referred to as a bypass bore). The accessory sleeve 172 is in fluid communication with the manifold volume 76 that is primarily defined by the trunk 80.

The fluid characterization module 68 is configured to be removably positioned through the accessory opening 111 and supported within the accessory sleeve 172. A tray 180 may be provided to facilitate the removable positioning of the fluid characterization module 68 within the accessory sleeve 172. In a most general sense, the tray 180 provides the module coupler to which the module housing 72 of the fluid characterization module 68 is coupled. With the tray 180 positioned within the accessory sleeve 172, the fluid characterization module 68 is in optical communication with the suction path, and in particular the inflow of the fluid through the first inlet fitting 88a. The tray 180 includes sides 182, and a base portion 184 coupled to the sides 182 to collectively define a cavity. The fluid characterization module 68 may be coupled to the base portion 184 and/or disposed within the cavity. More particularly, the fluid characterization module 68 may include a printed circuit board (PCB) assembly 186 sized and shaped to an opening of the cavity defined by the module housing 72.

The tray 180 may also include a sealing member 188 adapted to be in sealing engagement with the accessory opening 111 when the tray 180 is within the accessory sleeve 172 to facilitate maintaining the suction path through the manifold 66. The sealing member 188 includes a resiliently flexible portion 190 between upper and lower regions 192. With inputs to control members 194, the flexible portion 190 is configured to resiliently and pivotably move at least a portion of the sealing member 188 away from the accessory opening 111 to provide "bleeding" of the suction path as described in commonly-owned International Publication No. WO2019/0222655, published November 21, 2019.

The fluid characterization module 68 includes the sensor assembly 70 that is configured to be removably inserted through the accessory opening 111 and positioned within the accessory sleeve 172. The sensor assembly 70 includes the emitters 96, 97 are configured to emit energy, and the sensors 98, 99 are configured to detect the emitted energy. The emitters 96, 97 and the sensors 98, 99 are positioned opposite the suction path. For example, the fluid characterization module 68 includes a recess, an aperture, or other type of void through which the suction path is directed. For example, as best shown in FIG. 16, the detection window 94 may be comprised of a clear tube disposed within the accessory sleeve 113 or another component of the tray 180. At least one of the emitters 96, 97 is positioned on one side of the detection window 94, and at least one of the sensors 98, 99 is positioned on the other side of the detection window 94. As best shown in FIG. 17, the emitters 96, 97 may be coupled to and positioned on opposing sides of the PCB assembly 186. In one example, the first emitter 96 is positioned opposite a recess of to the PCB assembly 186 and one of the sensors 98, and the second emitter 97 is positioned opposite the recess and the other one of the sensors 99. The first sensor 98 detects the transmitted light from the first emitter 96 as well as the scattered light from the second emitter 97. The second sensor 99 detects the transmitted light from the second emitter 97 as well as the scattered light from the first emitter 96. The four measurements - two of transmitted light and two of scattered light - are values provided to the controller or processor 60 to execute an algorithm to determine the concentration of blood of the fluid. It is understood that the aforementioned alternative arrangement in which there are two (or three) emitters and four sensors may be included on the implementation of the fluid characterization module 68 disposed on the tray 180.

As appreciated from the utilization of visible light and infrared light, the sensors 98, 99 may have high sensitivity in both the visible and infrared regions of the electromagnetic spectrum. One suitable sensor is the OSRAM SFH3310 phototransistor, which is not only optimized for detecting visible light but also maintains approximately thirty-five percent of its maximum sensitivity within the infrared region. It is noted that a magnitude of the infrared light being emitted by the second emitter 97 is relatively larger than the scattered green light, and thus the reduction in sensitivity of the sensors 98, 99 in the infrared region of the electromagnetic spectrum should not compromise performance of the sensors 98, 99.

The fluid characterization module 68 may further include at least one integrated circuit. In one implementation, fluid characterization module 68 includes an LED driver integrated circuit 196, a photosensor integrated circuit 198, and a microcontroller 200 in communication with the LED driver integrated circuit 196 and the photosensor integrated circuit 198. The LED driver integrated circuit 196 is configured to supply current to drive the first and second emitters 96, 97. The photosensor integrated circuit 198 is configured to transmit the signal generated by the sensors 98, 99 to the microcontroller 200 (or to the controller 60 or another processor). The microcontroller 200 is configured to convert the signals to the aforementioned values provided to the algorithm to determine the concentration of blood of the fluid.

The fluid characterization module 68 may include a communication module 202 (see FIG. 27), such as a transceiver. The communication module 202 may be a component of the microcontroller 200. In one example, the communication module 202 utilizes Bluetooth low energy protocol to wirelessly transmit data. The data may be transmitted to the medical waste collection system 40, a mobile device with appropriate software, or any other suitable electronic device for assessing blood loss of the patient.

The fluid characterization module 68 may further include a battery 204, and a battery management integrated circuit 206. The LED driver integrated circuit 196, the photosensor integrated circuit 198, the microcontroller 200, and/or the communication module 202 may be in communication with the battery management integrated circuit 206 and configured to be powered by the battery 204 as regulated by the battery management integrated circuit 206. In one implementation, the battery 204 may be rechargeable. For example, the battery 204 may be recharged on a charging station, or a charging port integrated with the medical waste collection system 40. The battery management integrated circuit 206 is configured to manage charging and monitoring of the power level of the battery 204. For instance, when the battery 204 is low, the battery management integrated circuit 206 may be configured to send an alert to be displayed on the control panel 58 or another electronic device.

Referring now to FIG. 18, another implementation of the fluid characterization module 68 is shown in which the module housing 72 is configured to be removably coupled to the head 78 of the manifold 66. The manifold 66 includes a plurality of inlet fittings 88a, 88b, 88c, 88d. Four inlet fittings are shown, but more or less are contemplated. At least one of the inlet fittings 88a, 88b, 88c, 88d may be optically clear. The module housing 72 includes at least one opening sized to receive at least one of the inlet fittings 88a, 88b, 88c, 88d so as to couple the fluid characterization module 68 to the manifold 66. The illustrated implementation shows a first and second of the inlet fittings 88a, 88b extending through openings of the module housing 72. The suction tube(s) may be coupled to the first and second inlet fittings 88a, 88b distal to the module housing 72 such that the module housing 72 is positioned between the suction tube(s). With the manifold 66 removably positioned within the receiver 62, the suction path is established from the suction tube(s), through the first and second inlet fittings 88a, 88b and the manifold volume to the receiver 62 and waste container 46 of the medical waste collection system 40. Therefore, the fluid characterization module 68 is in optical communication with the suction path through the first and second inlet fittings 88a, 88b that are optically clear. The fluid characterization module 68 may be electronically coupled to the medical waste collection system 40 with a dongle (not shown).

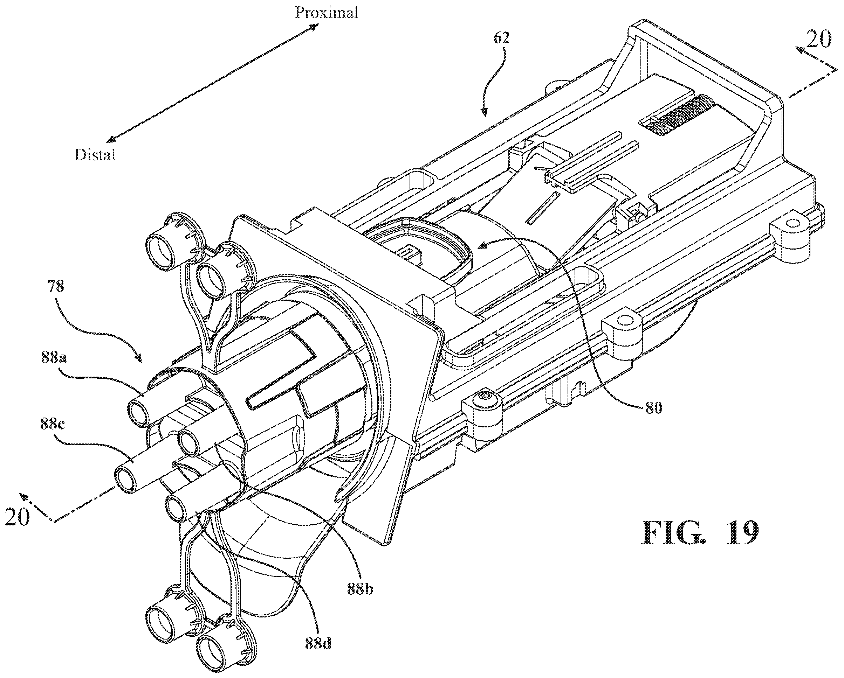

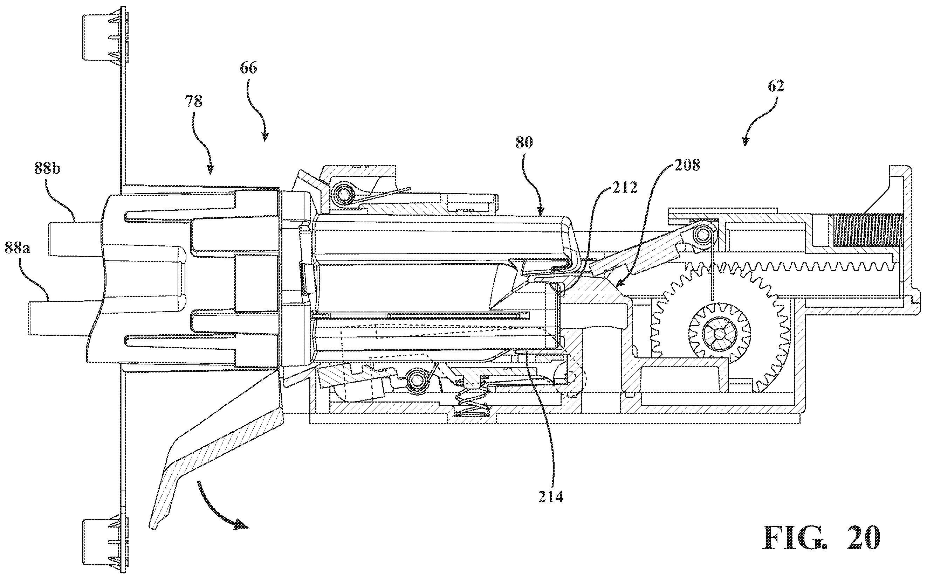

In certain implementations, the manifold 66 is disposable after each use and provides a sterile barrier between the fluid and the medical waste collection system 40. Likewise, the detection window 94 of the manifold 66 provides a sterile and liquid barrier between the fluid and the electronic components of the fluid characterization module 68. In such arrangements, the fluid characterization module 68 may be a capital component that is configured to be reused, whereas the manifold 66 may be a disposable component that is configured to be discarded after use. Therefore, it may be desirable to integrate the fluid characterization module 68 with or within components of the medical waste collection system 40. Referring now to FIGS. 19-22, the fluid characterization module 68 may be integrated with the receiver 62. In particular, the fluid characterization module 68 may be disposed on an inlet mechanism 208 of the receiver 62. The inlet mechanism 208 includes a suction fitting 210 defining a suction inlet and configured to penetrate the seal 84 to be at least partially positioned within the first leg 158 of the manifold 66, as best shown in FIG. 20. With the suction fitting 210 penetrating the seal 84, sealed fluid communication is provided between the manifold volume 76 and the receiver 62.

The inlet mechanism 208 may include a first support element 212 and a second support element 214. The first and second support elements 212, 214 may be configured to facilitate positioning the manifold 66 within the receiver 62 and supporting the manifold 66 in the fully inserted operative position. The first and second support elements 212, 214 may be arcuate in shape and contoured to the first leg 158. Further, the first and second support elements 212, 214 may be spaced apart from the suction fitting 210 by a distance at least equal to a thickness of the first leg 158. A depth of the space between the first and second support elements 212, 214 and the suction fitting 210 may be less than or equal to a depth of the void 162. With the manifold 66 is inserted into the receiver 62 in the fully inserted operative positive, the first support element 212 is seated or nestled within the void 162 with the seal 84 in engagement the suction fitting 210. The first support element 212 may further support the manifold 66 to minimize movement of the manifold 66 relative to the receiver 62 when in the fully inserted operative position.

The inlet mechanism 208 is movable within the receiver 62 to prevent fluid communication between the vacuum source 48 and the manifold 66 unless the manifold 66 is in the fully inserted operative position. In particular, the inlet mechanism 208 may be movable in the proximal-to-distal directions. As the manifold 66 is being moved proximally towards the fully inserted operative position, the inlet mechanism 208 translates distally, and as the manifold 66 is moved distally away from the fully inserted operative position, for example, during removal of the manifold 66, the inlet mechanism 208 translates proximally out of alignment with the receiver outlet. Finally, when the manifold 66 is in the fully inserted operative position, the suction outlet and the receiver outlet are aligned (see FIG. 20) to provide fluid communication between the manifold 66 and the waste container 46.

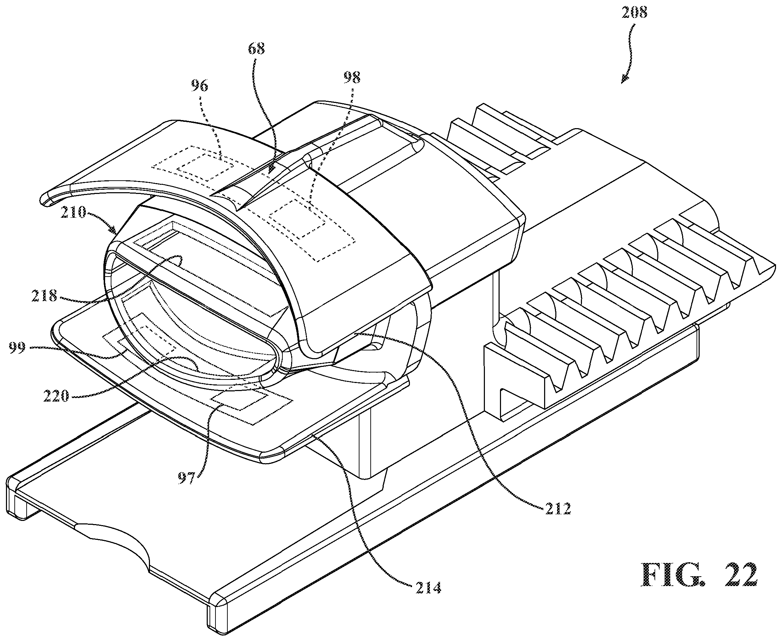

Owing to the inlet mechanism 208 defining a portion of the suction path in the present implementation, it is a particularly suitable location for integration with the fluid characterization module 68. FIG. 22 shows the emitters 96, 97 and the sensors 98, 99 coupled to the inlet mechanism 208. In particular, the first emitter 96 is coupled to the first support element 212, the second emitter 97 is coupled to the second support element 214, the first sensor 98 is coupled to the first support element 212, and the second sensor 99 is coupled to the second support element 214. The inlet mechanism 208 may further accommodate the LED driver integrated circuit 196, the photosensor integrated circuit 198, and the microcontroller 200. In the present implementation, the fluid characterization module 68 and its electronic components may be powered by a power source of the medical waste collection system 40, and therefore a battery may not be provided.

The suction fitting 210 may define a first window 218 and a second window 220. The first and second windows 218, 220 are configured to provide optical communication between the first emitter 96 and first sensor 98 on the first support element 212 and the second emitter 97 and the second sensor 99 on the second support element 214. The first and second windows 218, 220 of the inlet mechanism 208 are configured to be further aligned with first and second windows 222, 224 of the manifold 66 with the manifold 66 in the fully inserted operative position. In other words, the manifold 66 may include the detection window 94, which itself is formed from the first and second windows 222, 224. The first and second windows 222, 224 may be optically clear. Referring now to FIG. 21, the first leg 158 of the trunk 80 may define the first window 222 on an upper aspect 226 of the first leg 158, and the second window 224 on a lower aspect 228 of the first leg 158. With the first and second windows 222, 224 on the first leg 158, the catch 154 and the first window 222 are separated by the void 162. The first and second windows 222, 224 are positioned below the second leg 160 when the manifold 66 is oriented for insertion into the receiver 62. Further, the first and second windows 222, 224 may be positioned distal to the rim 164, distal to the proximally-directed surfaces 166 of the arms 148, distal to the proximally-directed surface 170 of the spine 152, distal to the distally-directed surfaces 168 of the catches 154, and proximal to the distally-directed surfaces of the lock elements 150.

With the manifold 66 in the fully inserted operative position, optical communication is provided between the emitters 96, 97 and the sensors 98, 99. In particular, light emitted from the first emitter 96 passes through the first window 218 of the inlet mechanism 208, the first window 222 of the manifold 66, the first leg 158 including the suction path, the second window 224 of the manifold 66, the second window 220 of the inlet mechanism 208, to the first sensor 98. Likewise, light emitted from the second emitter 97 passes through the second window 220 of the inlet mechanism 208, the second window 224 of the manifold 66, the first leg 158 including the suction path, the first window 222 of the manifold 66, the first window 218 of the inlet mechanism 208, to the second sensor 99. During operation of the medical waste collection system 40 with the manifold 66 in the fully inserted operative position, the fluid passes through the first leg 158 towards and through the seal 84, and therefore, the transmitted light and the scattered light previously described may be detected with the first and second sensors 98, 99 and the four values are provided to the algorithm. Moreover, the arrangement results in the fluid only contacting the manifold 66 and not the fluid characterization module 68, thereby limiting fouling and/or need to service or clean the inlet mechanism 208 internal to the medical waste collection system 40.

In certain implementations, a radiofrequency identification (RFID) tag 216 may be coupled to the manifold 66 and positioned to be detected by a sensor (*e.g.,* a data reader) of the medical waste collection system 40. Referring to FIGS. 3-6, 16, 18 and 21, the RFID tag 216 may be disposed on an upper wall or upper aspect of the trunk 80, and it should be understood that the remaining illustrations of the manifold 66 may similarly include the RFID tag 216. More particularly, the RFID tag 216 may be at least partially positioned on the body portion 156, and/or the RFID tag 216 may be at least partially positioned on the second leg 160. The RFID tag 216 may be configured to be detected by the data reader when the manifold 66 is in the first, second, third, and/or fully inserted operative positions. Should an article be incapable of being inserted to the fourth or fully inserted operative position for reasons previously described, no data communication is established between the RFID tag 216 and the reader, and the controller 60 may prevent operation of the medical waste collection system 40. In certain implementations, the RFID tag 216 may include memory storing data for determining whether the manifold 66 is usable with the medical waste collection system 40. The RFID tag 216 transmits the data from its memory to the data reader, and the controller 60 of the medical waste collection system 40 performs a consequent action. For example, the medical waste collection system 40 authenticates the manifold 66, and if successful, the medical waste collection system 40 may be operated as intended. In certain implementations, the memory of the RFID tag 216 may store calibration data for the emitters 96, 97 and/or the sensors 98, 99. With the authentication being successful, the calibration data is provided to the processor 60 to accurate quantification of the concentration of blood within the fluid.

As mentioned, quantifying blood concentration of the fluid from the patient may then facilitate quantifying blood loss of the patient - or QBL analysis - which is a metric of particular importance to the attending medical personnel. To quantify the volume of blood loss, an amount of the fluid being collected should be determined. In one example, the product of the concentration of blood within the fluid and the volume of the fluid at least approximately equals the volume of blood loss. One exemplary manner in which the volume of collected fluid may be determined is by measuring the volume of the fluid within the waste container 46 of the medical waste collection system 40. A fluid measuring assembly 47 may be provided in which a float element configured to float on the fluid moves along a sensor rod. An interrogating signal is sent along the sensor rod, and a return signal is detected with the return signal being based on the position of the float element along the sensor rod. One suitable fluid measuring assembly 47 is disclosed the aforementioned United States Patent No. 7,612,898.

The fluid measuring assembly 47 may be in communication with the processor 60, and/or in wireless communication with another device including a processor. Another exemplary manner in which the volume of collected fluid may be determined is by measuring a flow rate of the collected fluid over a known period of time. A flow rate sensor (not shown) may be disposed at any suitable location within the suction path. The flow rate sensor may be in communication with the processor 60, and/or in wireless communication with another device. The flow rate sensor may be an ultrasonic sensor. Implementations utilizing the flow rate sensor may provide for real-time quantification and display of the blood loss on the control panel 58 or another electronic device.

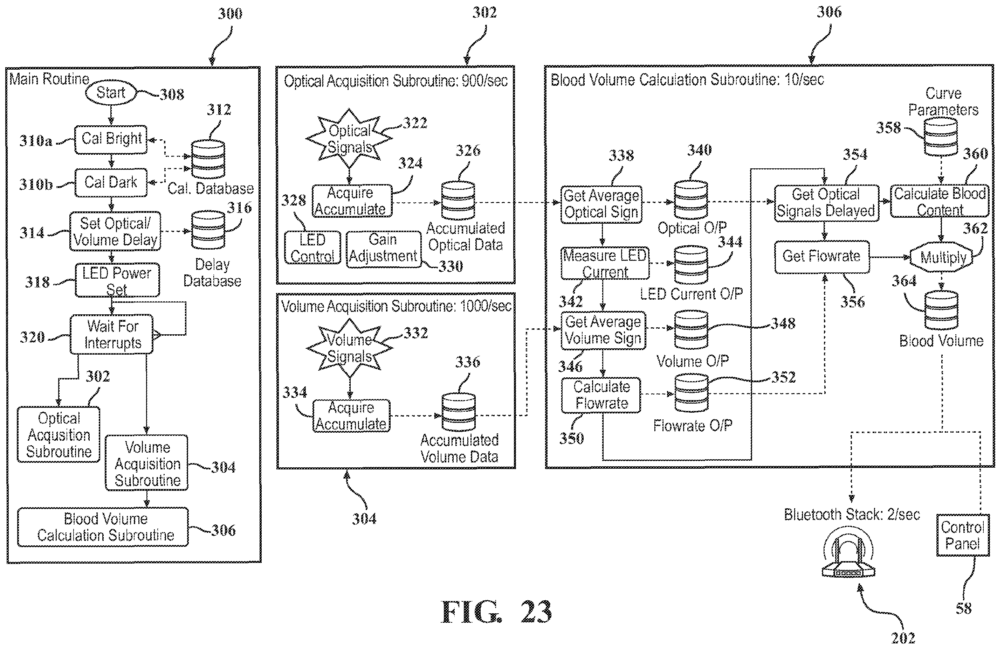

Referring now to FIG. 23, a schematic representation of workflow for real-time quantification of blood loss is shown in which a main routine 300 includes an optical acquisition subroutine 302, a volume acquisition subroutine 304, and a blood volume calculation subroutine 306. The main routine 300 starts a step 308. Step 308 may include the user initiating operation of the medical waste collection system 40 with the manifold 66 removably inserted into the receiver 62. Step 308 may further include the data reader of the medical waste collection system 40 detecting the RFID tag 216 disposed on the manifold 66 in which data transmitted to the data reader reflects that the manifold 66 includes the fluid characterization module 68. In other words, the medical waste collection system 40 identifies the manifold 66 as of the type used for determining blood loss (other manifolds may not have such capabilities), and thus the main routine 300 should start. Alternatively, step 308 may include the user selecting on the control panel 58 that the QBL analysis is desired.

At steps 310a, 310b, the emitters 96, 97 and/or the sensors 98, 99 may be calibrated to nominalize optical readings. The power output from the emitters 96, 97, sensitivity of the sensors 98, 99, and the optical clarity of the detection window 94 may drift over time. For example, the drift may be secondary to aging, temperature changes, fouling, part tolerances, or the like. Steps 310a, 310b may be performed at power-up and/or a "quiet period" in which the medical waste collection system 40 is idle. In one implementation, the steps 310a, 310b include calibrating each of the infrared LED and visible-light LED. The LEDs are switched off, and after several seconds for thermal equalization, output from the sensors 98, 99 is stored as a "dark" calibration reading, d. The LEDs are switched and after several seconds for thermal equalization, output from the sensors 98, 99 is stored as a "bright" calibration reading, b. These values are stored in memory of the calibration data database 312. During system operation, any sensor value read, *s*, is then converted to an absorbance value, *A*, using the equation:

$$A = 2 - \log_{10}(\frac{s - d}{b - d})$$

The execution of steps 310a, 310b render all subsequent readings relative to the bright and dark calibration values. Further, converting to absorbance also transforms the optical readings from a fundamentally logarithmic domain to a linear domain that is easier to model. The resulting calibration data may be provided to a calibration data database 312.

Alternatively, the calibration data may have been previously stored and provided by the calibration data database 312. The calibration data database 312 may store calibration data for one or more models of emitters, one or more models of photodetectors. The model of emitters and sensors on a particular fluid characterization module 68 may be data transmitted from the RFID tag 216 to the data reader. The calibration data may be written to the calibration data database 312. Steps 310a, 310b are optional.

At step 314, the optical signal delay may be set. Additionally or alternatively, the delay of volume signals may be set at step 314. Because there is a physical distance between the sensor assembly 70 and the fluid measuring assembly 47, there is a delay from when the sensor assembly 70 measures the properties of the fluid, and when that same fluid enters the waste container 46 and is measured by a volume change with the fluid measuring assembly 47. Further, because the blood concentration and collected fluid volume are utilized together to calculate the volume of blood loss, the two signals may be synchronized. In one implementation, the optical signal is delayed before multiplying it by the volume signal. The resulting delay data may be provided to a delay database 316. Step 314 may be optional. In another implementation, the delay is updated based on the calculated concentration of blood. In such an implementation, step 314 may be considered an initial delay, but thereafter the delay is continuously adjusted the delay based on the calculated percentage of blood. This may advantageously improve accuracy by accounting for high percentages of blood moving slower through the system, and therefore require a longer delay value.

After step 310, the optical acquisition subroutine 302, the volume acquisition subroutine 304, and the blood volume calculation subroutine 306 may be executed. In an exemplary implementation, the subroutines 302, 304, 306 are executed simultaneously. With continued reference to FIG. 23, the optical acquisition subroutine 302 includes step 320 of waiting for interrupts. The main routine 300 may be idle until notifications (interrupts) from the optical acquisition subroutine 302 and volume acquisition subroutine 304 that additional data has been generated. The main routine 300 uses the data to determine the blood volume for a given period. The main routine 300 then goes idle until the next interrupt. The optical acquisition subroutine 302 may further include step 322 of generating the optical signals. The optical signals are generated by the emitters 96, 97 emitting the light energy, for example, the visible light and the infrared light. The optical acquisition subroutine 302 includes step 324 of acquiring the optical signals. The optical signals are acquired by the sensors 98, 99, in particular the transmitted light and the scattered light for each of the visible light and the infrared light. Step 324 may include accumulating the optical signal data, and transmitted the optical signal data to an optical signal database 326. The optical acquisition subroutine 302 includes optional step 328 of controlling the emitters 96, 97, and/or optional step 330 of gain adjustment to be described in greater detail. Step 328 includes monitoring the current going through the emitters 96, 97. If the current increases or decreases (for instance due to some external influence such as a change in temperature), the control signal to the LED driver integrated circuit 196 is adjusted to compensate. The optical acquisition subroutine 302 may be executed at a sampling rate within the range of approximately 800 samples per second (sam/sec) to 1000 sam/sec, more particularly within the range of approximately 875 sam/sec to 925 sam/sec, and even more particularly at approximately 900 sam/sec. The conversion of the optical signals may occur at 10000 times/sec, and the filtered results of may be stored at 900 times/sec.

The volume acquisition subroutine 304 includes step 332 of generating volume signals for measuring of the volume of the fluid in the waste container 46, and step 334 of acquiring the signals. As previously described, the fluid measuring assembly 47 may determine the volume of collected fluid within the waste container 46, and/or the flow rate sensor may measure the flow rate of the fluid in the suction path for determining the volume of collected fluid. The determined volume is provided as volume data, and step 334 may include accumulating and transmitting the volume data to a volume data database 336. The volume acquisition subroutine 304 may be executed at a sampling rate within the range of approximately 900 calculations per second (calc/sec) to 1100 calc/sec, more particularly within the range of approximately 975 calc/sec to 1025 calc/sec, and even more particularly at approximately 1000 calc/sec.

The blood volume calculation subroutine 306 includes step 338 of receiving the accumulated optical signal data from the optical signal database 326, and calculating an average optical signal. The average optical signal may be provided to an optical output (O/P) database 340. The optical output is the average optical data from the sensors 98, 99 over the last time period (e.g., 1/10th second). Step 346 is similar but for the volume data. The volume data is then used to calculate the flowrate at step 350, in particular a difference between a newest volume measurement and an immediately previous volume measurement. The flowrate data may be provided to a flowrate O/P database 352. The delayed optical data is determined at step 354 and the data is converted to a percent concentration of blood.

This may be indicative of the blood concentration within the fluid the last time period (e.g., 1/10th second). The flow rate is obtained at step 356, and multiplied to the blood concentration to determine the volume of blood loss. The blood volume calculation subroutine 306 may be executed at a calculation rate within the range of approximately 5 calculations per second (calc/sec) to 15 calc/sec, more particularly within the range of approximately 8 calc/sec to 12 calc/sec, and even more particularly at approximately 10 calc/sec.

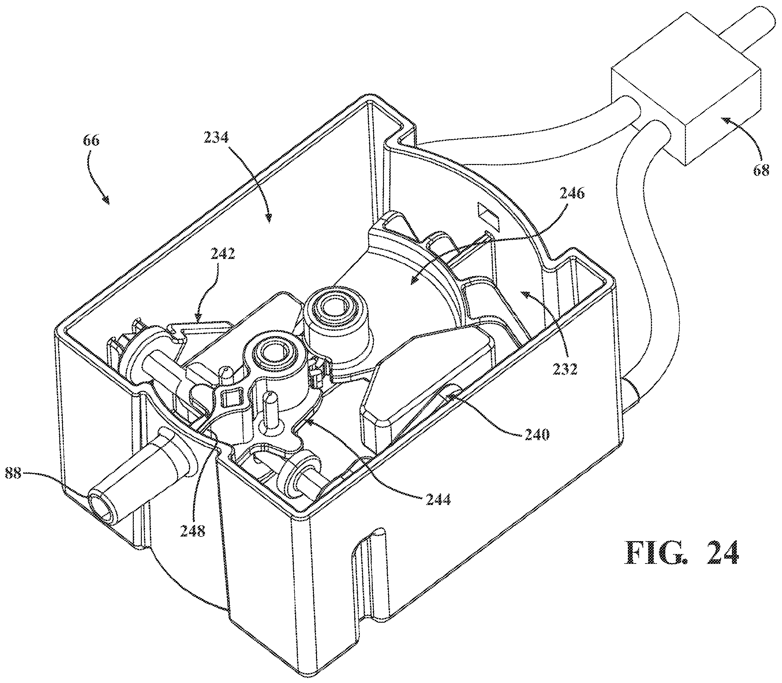

As mentioned, there may be a delay from when the sensor assembly 70 measures the optical properties of the fluid, and when that same fluid enters the waste container 46 to be measured the fluid measuring assembly 47. Referring now to FIGS. 23 and 24, another implementation of the manifold 66 is illustrated in which a volume of the fluid may be determined immediately prior to detecting the optical characteristics of the fluid with the fluid characterization module 68. The manifold 66 includes at least one barrier 230 dividing the manifold 66 into at least a first reservoir 232 and a second reservoir 234. The first reservoir 232 and the second reservoir 234 are not in fluid communication with one another. The inlet fitting 88 is in selective fluid communication with one of the first reservoir 232 and the second reservoir 234 in a manner to be described. The manifold 66 may include a first outlet fitting 236 defining a first outlet opening and a second outlet fitting 238 defining a second outlet opening each in fluid communication with a respective one of the first reservoir 232 and the second reservoir 234. Each of the first outlet fitting 236 and the second outlet fitting 238 are configured to receive an outlet suction tube such that the manifold 66 operates in an in-line configuration. The outlet suction tubes are coupled to the fluid characterization module 68. An adapter may be provided to merge the outlet suction tubes prior to the flow of fluid encountering the fluid characterization module 68.

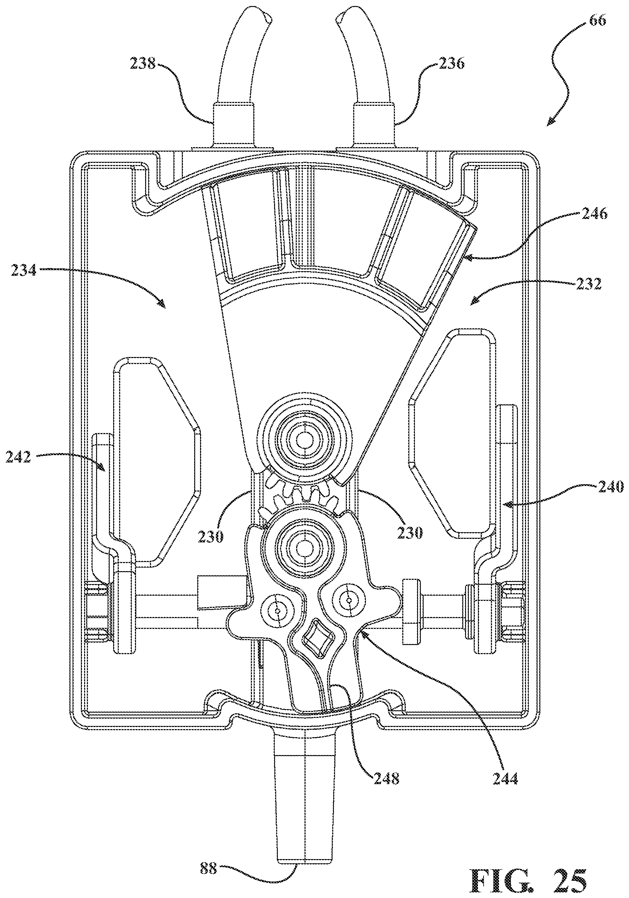

The manifold 66 includes a first fluid level assembly 240 and a second fluid level assembly 242 associated with a respective one of the first reservoir 232 and the second reservoir 234. FIG. 25 shows the first fluid level assembly 240 disposed within the first reservoir 232, and the second fluid level assembly 242 disposed in the second reservoir 234. The first fluid level assembly 240 and the second fluid level assembly 242 are configured to function as mechanically actuated valves to provide selective fluid communication between the inlet fitting 88 and one of the first reservoir 232 and the second reservoir 234, and further provide selective communication between one of the first reservoir 232 and the second reservoir 234 and its respective outlet fitting 238, 238. The first fluid level assembly 240 and the second fluid level assembly 242 include float elements coupled to a mechanism for pivoting a distal flow director 244 and a proximal flow director 246 coupled to the distal flow director 244. It is contemplated that electronic sensors may instead be provided to determine the respective fluid levels, and/or electronically actuated valves may instead be provided to perform the selective alternate of the suction path.

The first fluid level assembly 240 and the second fluid level assembly 242 are tuned to alternate the suction path between the first reservoir 232 and the second reservoir 234 at a predetermined or determinable fluid level. Owing to the dimensions of the manifold 66 being fixed, the suction path is effectively alternated between the first reservoir 232 and the second reservoir 234 at a known volume of fluid. Therefore, once the suction path is alternated, the known volume of fluid is suctioned through the fluid characterization module 68, thereby eliminating the aforementioned delay between optical measurements and volume measurements.

For example, FIG. 25 shows the manifold 66 in a first configuration in which the distal flow director 244 is positioned or angled such that a second barrier 248 directs the fluid into the second reservoir 234. The proximal flow director 246 is corresponding positioned or angled to block the second outlet opening and permit flow through the first outlet opening. As a vacuum is drawn on both of the outlet suction tubes, there is no vacuum on the second reservoir 234, yet there is vacuum on the inlet fitting 88 through the first reservoir 232. The inflow of fluid collects in the second reservoir 232, and the float element of the second fluid level assembly 242 rises correspondingly. Owing to the interconnection of the mechanisms, once the collected fluid and the float in the second reservoir 232 reaches the predetermined level, the distal flow director 244 and proximal flow director 246 have been shifted to the alternated positions. In other words, the manifold 66 is moved to a second configuration in which the distal flow director 244 is positioned or angled to direct the fluid into the first reservoir 232, and the proximal flow director 246 is positioned or angled to permit flow through the second outlet opening. In the second configuration, the vacuum begins to empty the second reservoir 234 through the fluid characterization module 68. The emitters 96, 97 and sensors 98, 99 detect the optical characteristic of the waste fluid being directed from the from the second reservoir 234 to the waste container 46. Perhaps simultaneously, additional waste fluid is accumulating in the first reservoir 232. The manifold 66 may be selectively alternated or toggled between the first and second configurations to repeat the process as many times as desired or necessary.

As previously described, the optical acquisition subroutine 302 includes the step 330 of gain adjustment. The gain adjustment acknowledges that blood is a highly effective at absorbing and scattering light, and therefore, as the concentration of blood increases, the amount of light passing through the blood decreases very quickly, thereby possibly leading to poor sensitivity and resolution of the sensors 98, 99 at high concentrations. For example, if a high gain of the sensors 98, 99 was selected, the sensors 98, 99 would be saturated when blood concentration levels are low, thereby possibly leading to poor sensitivity and resolution at low concentrations. To overcome this problem, the fluid characterization module 68 advantageously provides for on-the-fly adjustment the gain of one or both of the sensors 98, 99. Thus, as the concentration of blood increases, the gain of the sensors 98, 99 may be increased. More particularly, as the light detected by the sensors 98, 99 falls below a predetermined transmissivity threshold, the gain of the sensors 98, 99 is increased. Conversely, as the concentration of blood decreases, the gain of the sensors 98, 99 may be decreased. More particularly, as the light detected by the sensors 98, 99 rises above the predetermined transmissivity threshold (or another predetermined transmissivity threshold), the gain of the sensors 98, 99 is decreased.

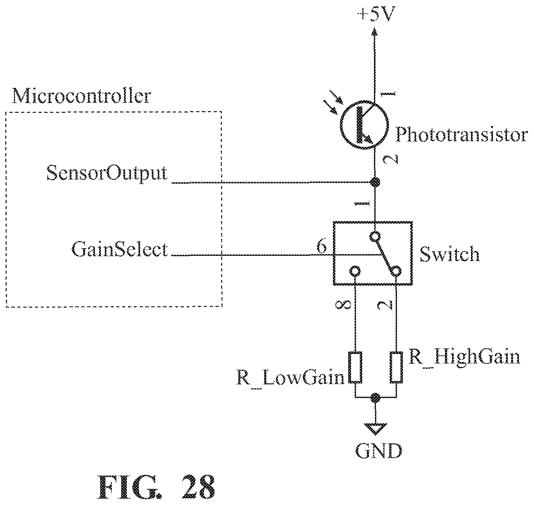

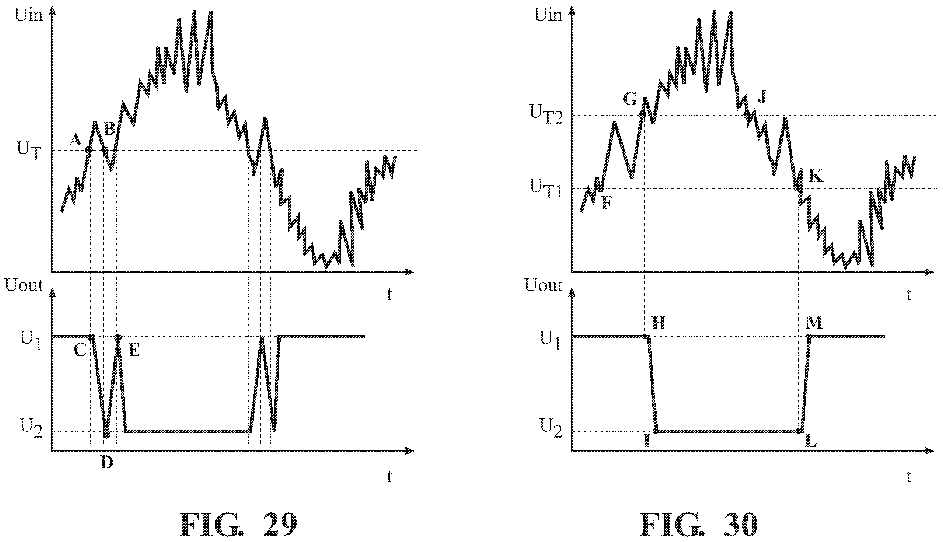

In one implementation, the photodetector detects a first light transmissivity of the fluid at a first gain level and generates a transmissivity signal. The transmissivity signal is transmitted to the controller or processor 60). The processor 60 changes the first gain level to a second gain level based on the transmissivity signal. The controller or processor 60 determines the concentration of blood based on the transmissivity signal and at least one of the first gain level and the second gain level. The second gain level may be greater than or less than the first gain level. For example, and with reference to FIG. 29 showing light transmissivity ($U_{IN}$) and gain ($U_{OUT}$) over time ($t$), the gain is at a first gain level ($U_1$) and light transmissivity initially increases to exceed the predetermined transmissivity threshold ($U_T$) at Point A. The controller or processor 60 is configured to adjust the gain level from the first gain level at Point C to the second gain level ($U_2$) at Point D. The first gain level and/or the second gain level may be stored as a function based on transmissivity or another sensed parameter of the fluid. Thereafter, the concentration of blood further decreases, but then increases such that the light transmissivity decreases until it falls below the predetermined transmissivity threshold at Point B. The controller or processor 60 is configured to adjust the gain level from the second gain level at Point D to the first gain level at Point E. The adjustment of the gain level between the first and second gain levels may occur at each instance the light transmissivity passes through the predetermined transmissivity threshold. It is understood that there may be more than one predetermined transmissivity threshold such that more than two different gain levels may be realized. Further, by employing an analogue switch controlled by a microcontroller, and multiple resistors, the gain can be selectively changed as required, as shown in FIG. 28.

Should the light transmissivity fluctuate across the predetermined transmissivity threshold, the gain may be adjusted repeated, perhaps excessively. A level of hysteresis may be included to avoid excessive switching of the gain level. An exemplary solution to provide for the aforementioned gain adjustment with less "noise" is shown in FIG. 30 in which first and second predetermined transmissivity thresholds ($U_{T1}$, $U_{T2}$) are utilized. More particularly, the second predetermined transmissivity threshold is greater than the first predetermined transmissivity threshold such that there is a higher threshold for increasing the gain level than for decreasing the gain level. For example, the gain is at the first gain level, and light transmissivity initially increases and exceeds the first predetermined transmissivity threshold at Point F. Because the decrease in the gain level is limited to the measured light transmissivity rising above or exceeding the second predetermined transmissivity threshold, the gain level is not adjusted at Point F. The plot shows the light transmissivity further increasing to exceed the first predetermined transmissivity threshold at Point G. The controller or processor 60 is configured to adjust the gain level from the first gain level at Point H to the second gain level at Point I. Thereafter, the plot shows that the concentration of blood further increases, then decreases to be below the second predetermined transmissivity threshold at Point J. Because the increase in the gain level is limited to the measured light transmissivity being below the first predetermined transmissivity threshold, the gain level is not adjusted at Point J. The plot shows the light transmissivity further decreasing to be below the first predetermined transmissivity threshold at Point K. The controller or processor 60 is configured to adjust the gain level from the second gain level at Point L to the first gain level at Point M. It is understood that there may be more than two predetermined transmissivity thresholds such that more than three different gain levels may be realized.

It is contemplated that the sensor assembly 70 may have additional sensors that are selectively operated based on the detected light transmissivity. For example, one or more of sensors may be calibrated to low light transmissivity, and others calibrated to high light transmissivity. Certain sensors may be set to operate by default. Should the detected light transmissivity decrease below the predetermined transmissivity threshold, the controller 60 may selectively activate the sensor(s) calibrated to the low light transmissivity. Should the detected light transmissivity return or increase above the predetermined transmissivity threshold, the controller 60 may selectively activate the sensor(s) calibrated to the high light transmissivity. Additionally or alternatively, the brightness of the emitter(s) 96, 97 may be adjusted based on the detected light transmissivity. For example, should the detected light transmissivity decrease below the predetermined transmissivity threshold, the controller 60 may increase the brightness of the light emitted from the emitters 96, 97 (or activate brighter emitters). Conversely, should the detected light transmissivity return or increase above the predetermined transmissivity threshold, the controller 60 may decrease the brightness of the light emitted from the emitters 96, 97 (or deactivate the brighter emitters).

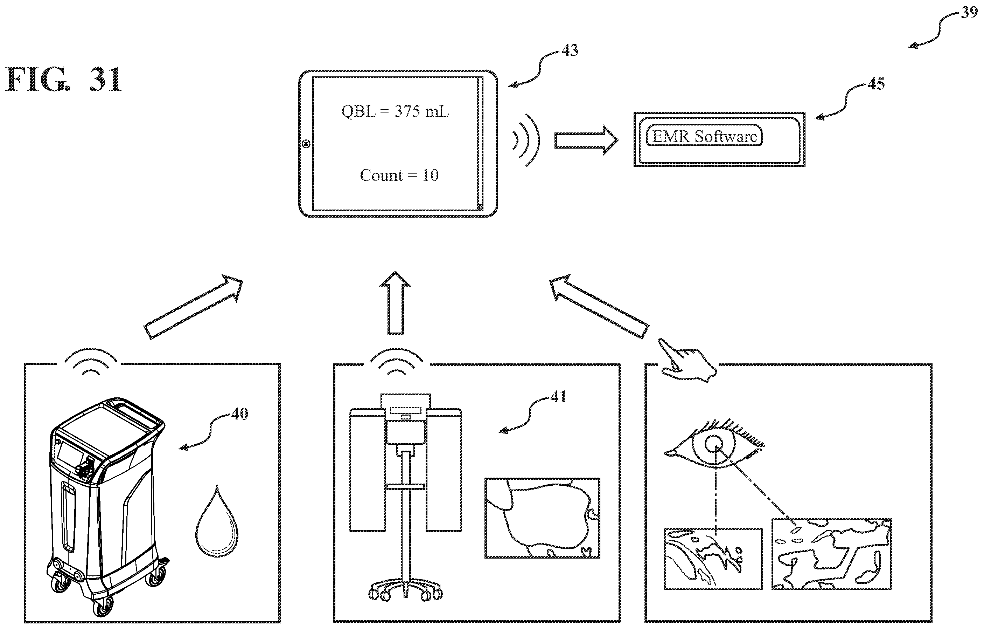

Referring now to FIG. 31, a blood management system 39 may include the medical waste collection system 40, a sponge system 41, and a user interface 43. The blood management system 39, in a most general sense, is to provide real-time quantification of patient blood loss leveraging the systems 40, 41, 43 to account for different manners in which blood may be presented in the operating suite. A compilation of the data from the systems 40, 41, 43 advantageously provides for real-time and accurate quantification of patient blood loss displayed on the user interface 43. The data may optionally be forwarded to the electronic medical record (EMR) 45 of the patient. The improved accuracy provides for more reliable visual and audible alarms that may be provided to the attending medical personnel in occurrences of excessive blood loss.

The blood management system 39 includes the medical waste collection system 40 that is described throughout the present disclosure and herein referred to by reference. The medical waste collection system 40 may separate blood from other bulk fluids with the subsequent volume measurement being used for blood loss volume calculations. Additionally or alternatively, the medical waste collection system 40 may receive an input from a user that only blood is being suctioned. For example, it is known to tare the volume of fluid following collection of amniotic fluid. The fluid characterization module 68 is either integrated with or removably coupled to the manifold 66, and/or integrated with the medical waste collection system 40 through any one or more of its implementations described herein.

The medical waste collection system 40 may perform the QBL analysis, and transmits the blood volume data wirelessly to the user interface 43. Additionally or alternatively, the user interface 43, another device such as a mobile device, or a remote server or the like, may receive the data described herein and execute the algorithm to perform the QBL analysis. The medical waste collection system 40 is in electronic communication with the user interface 43. Exemplary modes of electronic communication include Bluetooth low energy protocol, and a local area network (LAN) to which each the medical waste collection system 40 and the user interface 43 are wirelessly connected.

The sponge system 39 is configured to determining blood loss volume contained within absorbent articles, such as surgical sponges. One exemplary sponge system is sold under the tradename SurgiCount by Stryker Corporation (Kalamazoo, Mich.). The sponge system 31 includes a stand having on-board components for calculating a parameter of the blood loss. The stand includes one or more detection devices, and one or more mass measurement devices. In an exemplary implementation, the detection device is a code reader and the mass measurement device is a load cell.

In another implementation, the mass measurement device may be a container assembly for the absorbent articles with embedded load cell for weighing the absorbent articles. This container assembly may also include electronics configured to detect the absorbent articles within the container assembly. As the absorbent articles are detected, information is transmitted to the processor to identify them by part number and dry weight from a pre-programmed dataset.

At a time that blood loss volume data is indicated, a bag or other storage product can be physically supported on or by the mass measurement device. The bag may include a scannable code associated with its part number and its dry weight. As absorbent article(s) are introduced into the bag, a scannable code disposed on the absorbent article is scanned by the code reader. A database includes the part number and a dry weight of the absorbent article. The mass measurement device determines the total weight, and the dry weight(s) of the absorbent articles are subtracted to calculate an absorbed fluid weight. From the weight and known density of blood, absorbed blood loss volume may be calculated. The sponge system 41 is in electronic communication with the user interface 43, and the sponge system 41 may transmit the blood loss volume wirelessly or via a wired connection to the user interface 43.

In another implementation, accuracy of the dry weight may be improved by measuring mass of the bag or the absorbent articles during manufacturing in which the measured mass(es) are stored in the pre-programmed dataset of the RFID tag 216, for example. The measured mass may be based on a measured lot or pack average mass.

The user interface 43 functions as a hub for to provide acute patient information to the attending medical personnel. The volume of blood loss may be displayed on the control panel 58 and/or the user interface 43 in real-time throughout the procedure. The volume of blood loss may also be displayed as a graphical plot over the time since the procedure was initiated. In an exemplary implementation, the user interface 43 is a tablet with a touchscreen display for displaying all suitable information such as suctions blood loss volume, absorbed blood loss volume, alarms, warnings, and all other critical information. For example, the rate of blood loss may be used to trigger an alarm to warn the health care staff of a high rate of blood loss and thus the possibility of post-partum haemorrhage. The user interface 43 displays total patient blood loss by combining all relevant data. The alarms or warnings may be based on thresholds or guidelines wirelessly pushed to the user interface 43. The thresholds may be pre-determined by the manufacturer, implemented based on healthcare facility protocols, or acquired through clinical or other guidelines. The guidelines may be based on external clinical organizations, artificial intelligence decisions from clinical data mining, or other sources. Additional alerts could be generated based on blood loss rate. Moreover, the touchscreen display is configured to receive user inputs, in particular qualitative inputs from the attending medical personnel relevant to blood loss. The qualitative inputs may include an estimation of blood loss volume visualized on the floor or additional absorbent articles.

A volume of irrigation fluid used during the procedure, if known, may also be input to the touchscreen display. Additionally or alternatively, the irrigation fluids may be gravity-fed supported by a system capable of measuring and/or communicating the mass or volume of fluid used. An initial volume of the irrigation fluid may be entered, measured, and/or scanned. The irrigation system may include a load cell that measuring a current mass for calculation of the volume of irrigation fluid used. Additionally or alternatively, should an electronic pump be utilized to deliver the irrigation fluids, the electronic pump may generate and transmit data indicative of the volume of the irrigation fluid used.

## Claims

1. A manifold (66) for quantifying blood within fluid and configured to be removably inserted into a manifold receiver (62) of a medical waste collection system (40) that includes a vacuum source (52), the manifold (66) comprising:

   a housing (74) comprising a body portion (156) and an inlet fitting (88) configured to be removably coupled with a suction tube for drawing the fluid through the manifold (66) under influence of vacuum from the vacuum source (52); and
   a filter element (86, 130) disposed within the housing (74),
   wherein the housing (74) defines a manifold volume (76) and comprises a projection (90) defining a sump (100) below the manifold volume (76),
   **characterized in that**
   the housing (74) comprises a first leg (158) extending from the body portion (156) and comprising a rim (164) defining an outlet opening (82), and a second leg (160) extending from the body portion (156) and spaced apart from the first leg (158) to define a void (162), and **in that** at least a portion of the projection (90) is optically clear to comprise a detection window (94) configured to be positioned between an emitter (96, 97) and a detector (98, 99) of an optical sensor assembly (70).

2. The manifold (66) of claim 1, wherein the housing (74) further comprises a trunk (80) comprising the first leg (158) and the second leg (160), and a head (78) coupled to the trunk (80) and comprising the inlet fitting (88), wherein the head (78) comprises the detection window (94).

3. The manifold (66) of claim 1, wherein the projection (90) comprises a coupling feature configured to be removably coupled to a fluid characterization module (68) that includes the optical sensor assembly (70).

4. The manifold (66) of claim 3, wherein the coupling feature comprises at least one rail configured to slidably engage a slot (92) of the fluid characterization module (68).

5. The manifold (66) of any one of claims 1-4, wherein the projection (90) has a width of no greater than 19.05 mm.

6. The manifold (66) of any one of claims 1-4, wherein the filter element (86, 130) is a first filter element (86) disposed within the manifold volume (76), the manifold (66) further comprising a second filter element (130) disposed within the sump (100).

7. The manifold (66) of claim 6, further comprising a straw (136) comprising a first end (138) disposed near a base (134) of the sump (100), and a second end (140) disposed within the manifold volume (76).

8. The manifold (66) of claim 7, wherein the straw (136) extends through the second filter element (130).

9. The manifold (66) of any one of claims 1-8, further comprising a fluid director (104) disposed within the housing (74), wherein the fluid director (104) comprises geometries configured to provide a tortuous path to the fluid within the manifold (66).

10. The manifold (66) of claim 9, wherein the fluid director (104) comprises a barrier (106) positioned above the detection window (94) and defining a liquid inlet (102) configured to facilitate accumulation of the fluid within the housing (74) during which gas within the fluid separates from liquid within the fluid.

11. The manifold (66) of claim 10, wherein the fluid director (104) further defines a gas inlet (110) positioned above the liquid inlet (102).

12. The manifold (66) of claim 11, wherein the fluid director (104) further defines a fluid outlet (116) in communication with each of the liquid inlet (102) and the gas inlet (110).

13. The manifold (66) of any one of claims 9-12, wherein the filter element (86) is disposed closer to the outlet opening (82) relative to the fluid director (104).

14. The manifold (66) of any one of claims 9-13, wherein the fluid director (104) is disposed closer to the outlet opening (82) relative to the filter element (86).

15. The manifold (66) of any one of claims 1-14, further comprising a radiofrequency identification tag (216) disposed on the housing (74) and comprising memory storing data indicative of the manifold (66) as being of a type that for use with the optical sensor assembly (70) for quantifying blood loss.

**Patentansprüche**

1. Verteiler (66) zur Quantifizierung von Blut innerhalb eines Fluids und konfiguriert, um entnehmbar in eine Verteileraufnahme (62) eines Systems zur Sammlung medizinischer Abfälle (40) eingesetzt zu werden, das eine Vakuumquelle (52) umfasst, wobei der Verteiler (66) aufweist:

   ein Gehäuse (74), aufweisend einen Körperabschnitt (156) und einen Einlassanschluss (88), der konfiguriert ist, um entnehmbar mit einem Saugschlauch gekoppelt zu werden, um das Fluid unter Einfluss von Vakuum von der Vakuumquelle (52) durch den Verteiler (66) zu ziehen; und
   ein Filterelement (86, 130), das innerhalb des Gehäuses (74) angeordnet ist,
   wobei das Gehäuse (74) ein Verteilervolumen (76) definiert und einen Vorsprung (90) aufweist, der einen Sumpf (100) unterhalb des Verteilervolumens (76) definiert,
   **dadurch gekennzeichnet, dass**
   das Gehäuse (74) ein erstes Bein (158), das sich von dem Körperabschnitt (156) erstreckt und einen Rand (164) umfasst, der eine Auslassöffnung (82) definiert, und ein zweites Bein (160) umfasst, das sich von dem Körperabschnitt (156) erstreckt und von dem ersten Bein (158) beabstandet ist, um einen Hohlraum (162) zu definieren, und dadurch, dass
   zumindest ein Abschnitt des Vorsprungs (90) optisch klar ist, um ein Erfassungsfenster (94) aufzuweisen, das konfiguriert ist, um zwischen einem Emitter (96, 97) und einem Detektor (98, 99) einer optischen Sensoranordnung (70) positioniert zu werden.

2. Verteiler (66) nach Anspruch 1, wobei das Gehäuse (74) ferner einen Stamm (80), der das erste Bein (158) und das zweite Bein (160) aufweist, und einen Kopf (78) aufweist, der mit dem Stamm (80) gekoppelt ist und den Einlassanschluss (88) aufweist, wobei der Kopf (78) das Erfassungsfenster (94) aufweist.

3. Verteiler (66) nach Anspruch 1, wobei der Vorsprung (90) ein Kopplungsmerkmal aufweist, das konfiguriert ist, um entnehmbar mit einem Fluidcharakterisierungsmodul (68) gekoppelt zu sein, das die optische Sensoranordnung (70) umfasst.

4. Verteiler (66) nach Anspruch 3, wobei das Kopplungsmerkmal mindestens eine Schiene aufweist, die konfiguriert ist, um gleitend in einen Schlitz (92) des Fluidcharakterisierungsmoduls (68) einzugreifen.

**5.** Verteiler (66) nach einem der Ansprüche 1-4, wobei der Vorsprung (90) eine Breite von nicht mehr als 19,05 mm aufweist.

**6.** Verteiler (66) nach einem der Ansprüche 1-4, wobei das Filterelement (86, 130) ein erstes Filterelement (86) ist, das innerhalb des Verteilervolumens (76) angeordnet ist, wobei der Verteiler (66) ferner ein zweites Filterelement (130) aufweist, das innerhalb des Sumpfes (100) angeordnet ist.

**7.** Verteiler (66) nach Anspruch 6, ferner aufweisend ein Steigrohr (136), das ein erstes Ende (138), das nahe einer Basis (134) des Sumpfes (100) angeordnet ist, und ein zweites Ende (140) aufweist, das innerhalb des Verteilervolumens (76) angeordnet ist.

**8.** Verteiler (66) nach Anspruch 7, wobei sich das Steigrohr (136) durch das zweite Filterelement (130) erstreckt.

**9.** Verteiler (66) nach einem der Ansprüche 1-8, ferner aufweisend einen Fluidrichter (104), der innerhalb des Gehäuses (74) angeordnet ist, wobei der Fluidrichter (104) Geometrien umfasst, die konfiguriert sind, um einen gewundenen Pfad für das Fluid innerhalb des Verteilers (66) bereitzustellen.

**10.** Verteiler (66) nach Anspruch 9, wobei der Fluidrichter (104) eine Barriere (106) aufweist, die oberhalb des Erfassungsfensters (94) positioniert ist und einen Flüssigkeitseinlass (102) definiert, der konfiguriert ist, um eine Ansammlung des Fluids innerhalb des Gehäuses (74) zu erleichtern, während derer sich Gas innerhalb des Fluids von der Flüssigkeit innerhalb des Fluids trennt.

**11.** Verteiler (66) nach Anspruch 10, wobei der Fluidrichter (104) ferner einen Gaseinlass (110) definiert, der oberhalb des Flüssigkeitseinlasses (102) positioniert ist.

**12.** Verteiler (66) nach Anspruch 11, wobei der Fluidrichter (104) ferner einen Fluidauslass (116) in Verbindung mit jeweils dem Flüssigkeitseinlass (102) und dem Gaseinlass (110) definiert.

**13.** Verteiler (66) nach einem der Ansprüche 9-12, wobei das Filterelement (86) relativ zum Fluidrichter (104) näher an der Auslassöffnung (82) angeordnet ist.

**14.** Verteiler (66) nach einem der Ansprüche 9-13, wobei der Fluidrichter (104) relativ zum Filterelement (86) näher an der Auslassöffnung (82) angeordnet ist.

**15.** Verteiler (66) nach einem der Ansprüche 1-14, ferner aufweisend eine Radiofrequenz-Erkennungsmarke (216), die am Gehäuse (74) angeordnet ist und einen Speicher aufweist, der Daten speichert, die den Verteiler (66) als einen Typ kennzeichnen, der zur Verwendung mit der optischen Sensoranordnung (70) zur Quantifizierung von Blutverlust bestimmt ist.

**Revendications**

**1.** Collecteur (66) destiné à quantifier le sang présent dans un fluide et conçu pour être inséré de manière amovible dans un récipient de collecteur (62) d'un système de collecte de déchets médicaux (40) comprenant une source de vide (52), le collecteur (66) comprenant:

un boîtier (74) comprenant une partie corps (156) et un raccord d'entrée (88) conçu pour être raccordé de manière amovible à un tube d'aspiration afin d'aspirer le fluide dans le collecteur (66) sous l'effet du vide provenant de la source de vide (52); et
un élément filtrant (86, 130) disposé à l'intérieur du boîtier (74),
dans lequel le boîtier (74) définit un volume de collecteur (76) et comporte une saillie (90) définissant un bac (100) situé sous le volume de collecteur (76),
**caractérisé en ce que**
le boîtier (74) comprend un premier segment (158) s'étendant à partir de la partie corps (156) et comportant un rebord (164) délimitant une ouverture de sortie (82), ainsi qu'un deuxième segment (160) s'étendant à partir de la partie corps (156) et espacée du premier segment (158) de manière à délimiter un espace vide (162), et **en ce qu'**
au moins une partie de la saillie (90) est optiquement transparente pour former une fenêtre de détection (94) conçue pour être positionnée entre un émetteur (96, 97) et un détecteur (98, 99) d'un ensemble capteur optique (70).

**2.** Collecteur (66) selon la revendication 1, dans lequel le boîtier (74) comprend en outre un corps (80) comportant le premier segment (158) et le deuxième segment (160), ainsi qu'une tête (78) reliée au corps allongé (80) et comportant le raccord d'entrée (88), la tête (78) comportant la fenêtre de détection (94).

**3.** Collecteur (66) selon la revendication 1, dans lequel la saillie (90) comprend un élément d'accouplement conçu pour être accouplé de manière amovible à un module de caractérisation de fluide (68) qui comprend l'ensemble capteur optique (70).

**4.** Collecteur (66) selon la revendication 3, dans lequel l'élément d'accouplement comprend au moins un rail conçu pour s'insérer en coulissement dans une fente (92) du module de caractérisation de fluide (68).

**5.** Collecteur (66) selon l'une quelconque des revendications 1 à 4, dans lequel la saillie (90) présente une largeur ne dépassant pas 19,05 mm.

**6.** Collecteur (66) selon l'une quelconque des revendications 1 à 4, dans lequel l'élément filtrant (86, 130) est un premier élément filtrant (86) disposé à l'intérieur du volume de collecteur (76), le collecteur (66) comprenant en outre un deuxième élément filtrant (130) disposé à l'intérieur du bac (100).

**7.** Collecteur (66) selon la revendication 6, comprenant en outre une tige (136) comportant une première extrémité (138) disposée à proximité d'une base (134) du bac (100), et une deuxième extrémité (140) disposée à l'intérieur du volume de collecteur (76).

**8.** Collecteur (66) selon la revendication 7, dans lequel la tige (136) traverse le deuxième élément filtrant (130).

**9.** Collecteur (66) selon l'une quelconque des revendications 1 à 8, comprenant en outre un déflecteur de fluide (104) disposé à l'intérieur du boîtier (74), dans lequel le déflecteur de fluide (104) comporte des géométries conçues pour créer un trajet sinueux pour le fluide à l'intérieur du collecteur (66).

**10.** Collecteur (66) selon la revendication 9, dans lequel le déflecteur de fluide (104) comprend une barrière (106) placée au-dessus de la fenêtre de détection (94) et délimitant une entrée de liquide (102) conçue pour faciliter l'accumulation du fluide à l'intérieur du boîtier (74), au cours de laquelle le gaz contenu dans le fluide se sépare du liquide contenu dans le fluide.

**11.** Collecteur (66) selon la revendication 10, dans lequel le déflecteur de fluide (104) délimite en outre une entrée de gaz (110) située au-dessus de l'entrée de liquide (102).

**12.** Collecteur (66) selon la revendication 11, dans lequel le déflecteur de fluide (104) délimite en outre une sortie de fluide (116) en communication avec l'entrée de liquide (102) et l'entrée de gaz (110).

**13.** Collecteur (66) selon l'une quelconque des revendications 9 à 12, dans lequel l'élément filtrant (86) est plus proche de l'ouverture de sortie (82) par rapport au déflecteur de fluide (104).

**14.** Collecteur (66) selon l'une quelconque des revendications 9 à 13, dans lequel le déflecteur de fluide (104) est plus proche de l'ouverture de sortie (82) par rapport à l'élément filtrant (86).

**15.** Collecteur (66) selon l'une quelconque des revendications 1 à 14, comprenant en outre une étiquette d'identification par radiofréquence (216) disposée sur le boîtier (74) et comportant une mémoire stockant des données indiquant que le collecteur (66) est d'un type destiné à être utilisé avec l'ensemble capteur optique (70) pour quantifier la perte de sang.

FIG. 1

FIG. 2

FIG. 3

FIG. 4

FIG. 5

**FIG. 6**

FIG. 7

EP 4 243 890 B1

FIG. 8

**FIG. 9**

FIG. 10

**FIG. 11**

FIG. 12

**FIG. 13**

FIG. 14

**FIG. 15**

EP 4 243 890 B1

FIG. 16

FIG. 17

FIG. 18

Proximal

Distal

62

20

78

88a

88c

20

88b

88d

80

FIG. 19

42

FIG. 20

FIG. 21

FIG. 22

**FIG. 23**

FIG. 24

47

FIG. 25

FIG. 26

FIG. 27

FIG. 28

FIG. 30

FIG. 29

FIG. 31

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63112382
- US 20050171495 A
- WO 2007070570 A
- WO 2014066337 A
- WO 2016183290 A1
- US 2020324028 A1
- US 2020061255 A1
- US 2016367734 A1
- US 7621898 B
- US 201715284 B
- US 10105470 B
- US 7612898 B
- US 7615037 B
- WO 2020209898 A